# EUROPEAN PATENT SPECIFICATION

(11) **EP 2 582 721 B1**
(45) Date of publication and mention of the grant of the patent: **22.08.2018**
(21) Application number: 11796555.8
(22) Date of filing: 17.06.2011
(51) Int. Cl.: A61K 39/395, C07K 16/10, A61P 31/16

(54) **ANTIBODIES USEFUL IN PASSIVE INFLUENZA IMMUNIZATION**
ANTIKÖRPER ZUR PASSIVEN INFLUENZA-IMMUNISIERUNG
ANTICORPS UTILES DANS L'IMMUNISATION PASSIVE CONTRE LA GRIPPE

(30) Priority: 17.06.2010 US 355978 P; 15.02.2011 US 201161443103 P; 22.02.2011 US 201161445455 P
(43) Date of publication of application: 24.04.2013
(73) Proprietor: Trellis Bioscience, LLC, Menlo Park, CA 94025 (US)
(72) Inventor: KAUVAR, Lawrence, M., San Francisco, CA 94109 (US); ELLSWORTH, Stote, Palo Alto, CA 94301 (US); USINGER, William, Lafayette, CA 94549 (US); MCCUTCHEON, Krista, Maureen, Burlingame, CA 94010 (US); PARK, Minha, Brisbane, CA 94005 (US)
(74) Representative: V.O.
(86) International application number: PCT/US2011/040982
(87) International publication number: WO 2011/160083

(56) References cited:
- WO-A1-2010/022120
- WO-A1-2010/074656
- WO-A1-2011/117848
- WO-A2-2007/134327
- WO-A2-2008/028946
- WO-A2-2008/076379
- WO-A2-2009/079259
- WO-A2-2009/121004
- WO-A2-2010/010466
- WO-A2-2010/010467
- WO-A2-2012/045001
- US-A1- 2009 311 265
- US-A1- 2010 086 555
- US-B2- 7 696 330
- DAVIDE CORTI ET AL: "A Neutralizing Antibody Selected from Plasma Cells That Binds to Group 1 and Group 2 Influenza A Hemagglutinins", SCIENCE, AMERICAN ASSOCIATION FOR THE ADVANCEMENT OF SCIENCE, WASHINGTON, DC; US; THE INSTITUTION OF ELECTRICAL ENGINEERS, STEVENAGE, GB, vol. 333, no. 6044, 12 August 2011 (2011-08-12), pages 850-856, XP002689150, ISSN: 0036-8075, DOI: 10.1126/SCIENCE.1205669 [retrieved on 2011-07-28]
- PRABHU NAYANA ET AL: "Monoclonal antibodies against the fusion peptide of hemagglutinin protect mice from lethal influenza A virus H5N1 infection.", JOURNAL OF VIROLOGY MAR 2009, vol. 83, no. 6, March 2009 (2009-03), pages 2553-2562, XP002714198, ISSN: 1098-5514
- ZIEGLER T ET AL: "Type- and subtype-specific detection of influenza viruses in clinical specimens by rapid culture assay", JOURNAL OF CLINICAL MICROBIOLOGY, AMERICAN SOCIETY FOR MICROBIOLOGY, US, vol. 33, no. 2, 1 February 1995 (1995-02-01), pages 318-321, XP002541924, ISSN: 0095-1137
- KOSTOLANSKÝ F ET AL: "Antibody response to hidden epitope of influenza A haemagglutinin elicited by anti-idiotypic antibodies.", ACTA VIROLOGICA AUG 1994, vol. 38, no. 4, August 1994 (1994-08), pages 215-222, XP008165167, ISSN: 0001-723X
- EKIERT DAMIAN C ET AL: "Antibody Recognition of a Highly Conserved Influenza Virus Epitope", SCIENCE, AMERICAN ASSOCIATION FOR THE ADVANCEMENT OF SCIENCE, WASHINGTON, DC; US, vol. 324, no. 5924, 1 April 2009 (2009-04-01), pages 246-251, XP009144786, ISSN: 0036-8075
- DONIS R O ET AL: "Distinct lineages of influenza virus H4 hemagglutinin genes in different regions of the world", VIROLOGY, ELSEVIER, AMSTERDAM, NL, vol. 169, no. 2, 1 April 1989 (1989-04-01) , pages 408-417, XP026430699, ISSN: 0042-6822 [retrieved on 1989-04-01]
- STEEL ET AL.: 'Influenza Virus Vaccine Based on the Conserved Hemagglutinin Stalk Domain' MBIO vol. 1, no. 1, April 2010, pages 1 - 9, XP002676044
- KUBOTA-KOKETSU ET AL.: 'Broad neutralizing human monoclonal antibodies against influenza virus from vaccinated healthy donors.' BIOCHEMICAL AND BIOPHYSICAL RESEARCH COMMUNICATIONS vol. 387, 2009, pages 180 - 185, XP026378974
- BRIGHT ET AL.: 'Cross-Clade Protective Immune Responses to Influenza Viruses with H5N1 HA and NA Elicited by an Influenza Virus-Like Particle.' PLOS ONE vol. 3, no. E1501, January 2008, pages 1 - 14, XP009116084
- "Competitive inhibition", INTERNET CITATION, 13 September 2011 (2011-09-13), pages 1-5, XP002660444, Retrieved from the Internet: URL:http://en.wikipedia.org/wiki/Competiti ve_inhibition [retrieved on 2011-10-03]
- THROSBY MARK ET AL: "Heterosubtypic neutralizing monoclonal antibodies cross-protective against H5N1 and H1N1 recovered from human IgM+ memory B cells.", PLOS ONE 2008, vol. 3, no. 12, 2008, page e3942, ISSN: 1932-6203
- GERSHONI JONATHAN M ET AL: "Epitope mapping - The first step in developing epitope-based vaccines", BIODRUGS, ADIS INTERNATIONAL LTD, NZ, vol. 21, no. 3, 1 January 2007 (2007-01-01), pages 145-156, XP009103541, ISSN: 1173-8804, DOI: 10.2165/00063030-200721030-00002

### SEQUENCE LISTING

<110> TRELLIS BIOSCIENCE, INC.
   KAUVAR, Lawrence M.
   ELLSWORTH, Stote
   USINGER, William
   MCCUTCHEON, Krista Maureen
   PARK, Minha
<120> ANTIBODIES USEFUL IN PASSIVE INFLUENZA IMMUNIZATION
<130> 388512012840
<140> Not Yet Assigned
   <141> Concurrently Herewith
<150> US 61/445,455
   <151> 2011-02-22
<150> US 61/443,103
   <151> 2011-02-15
<150> US 61/355,978
   <151> 2010-06-17
<160> 84
<170> FastSEQ for Windows Version 4.0
<210> 1
   <211> 8
   <212> PRT
   <213> Artificial Sequence
<220>
   <223> synthetically constructed A/H3/HA0 consensus sequence
<400> 1
   Asn Val Pro Glu Lys Gln Thr Arg
    1               5
<210> 2
   <211> 11
   <212> PRT
   <213> Artificial Sequence
<220>
   <223> synthetically constructed A/H3/HA0 consensus sequence
<400> 2
   Gly Ile Phe Gly Ala Ile Ala Gly Phe Ile Glu
   1 5 10
<210> 3
   <211> 8
   <212> PRT
   <213> Artificial Sequence
<220>
   <223> synthetically constructed A/H1/HA0 consensus sequence
<400> 3
   Asn Ile Pro Ser Ile Gln Ser Arg
   1 5
<210> 4
   <211> 11
   <212> PRT
   <213> Artificial Sequence
<220>
   <223> synthetically constructed A/H1/HA0 consensus sequence
<400> 4
   Gly Leu Phe Gly Ala Ile Ala Gly Phe Ile Glu
   1 5 10
<210> 5
   <211> 8
   <212> PRT
   <213> Artificial Sequence
<220>
   <223> synthetically constructed B/HA0 consensus sequence
<400> 5
   Pro Ala Lys Leu Leu Lys Glu Arg
   1 5
<210> 6
   <211> 11
   <212> PRT
   <213> Artificial Sequence
<220>
   <223> synthetically constructed B/HA0 consensus sequence
<400> 6
   Gly Phe Phe Gly Ala Ile Ala Gly Phe Leu Glu
   1 5 10
<210> 7
   <211> 14
   <212> PRT
   <213> Artificial Sequence
<220>
   <223> synthetically constructed consensus sequence
<400> 7
   Arg Gly Ile Leu Phe Phe Gly Ala Ile Ala Gly Phe Leu Glu
    1               5                  10
<210> 8
   <211> 330
   <212> PRT
   <213> Homo sapiens
<220>
   <221> CHAIN
   <222> (1)...(330)
   <223> IgG1 heavy chain amino acid sequence of constant region
<400> 8
   Ala Ser Thr Lys Gly Pro Ser Val Phe Pro Leu Val Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 9
   <211> 121
   <212> PRT
   <213> Artificial Sequence
<220>
   <223> synthetically constructed MAB1 heavy chain amino acid sequence of variable domain
<400> 9
   Gln Val Gln Leu Gln Glu Ser Gly Pro Gly Leu Val Lys Pro Ser Glu
    1               5                  10                  15
   Thr Leu Ser Leu Ile Cys Arg Val Ser Gly Gly Ser Ile Ser Ser His
               20                  25                  30
   Tyr Trp Ser Trp Ile Arg Gln Pro Pro Gly Lys Gly Leu Glu Trp Ile
           35                  40                  45
   Gly Tyr Ile Ser Tyr Arg Gly Arg Ser Asn His Asn Pro Ser Leu Gly
       50                  55                  60
   Arg Arg Val Ser Met Ser Ile Asp Thr Ser Glu Asn Gln Phe Ser Leu
   65                  70                  75                  80
   Asn Leu Ser Ser Val Ile Ala Ala Asp Thr Ala Val Tyr Tyr Cys Ala
                   85                  90                  95
   Arg Asp Ala Thr Gly Ile Arg Glu Ile Asn Ala Leu Asp Ile Trp Gly
               100                 105                 110
   Gln Gly Thr Thr Val Thr Val Ser Ser
           115                 120
<210> 10
   <211> 121
   <212> PRT
   <213> Artificial Sequence
<220>
   <223> synthetically constructed MAB8 heavy chain amino acid sequence of variable domain
<400> 10
   Glu Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Phe Ser Thr Tyr
               20                  25                  30
   Thr Met Ser Trp Val Arg Gln Ala Pro Gly Gln Gly Leu Glu Trp Val
           35                  40                  45
   Ser Ser Ile Thr Arg Thr Ser Ser Asn Ile Tyr Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met His Ser Leu Arg Val Glu Asp Thr Ala Val Tyr Tyr Cys
                   85                  90                  95
   Ala Arg Ile Ser Gly Val Val Gly Pro Val Pro Phe Asp Tyr Trp Gly
               100                 105                 110
   Gln Gly Thr Leu Ile Thr Val Ser Ser
           115                 120
<210> 11
   <211> 118
   <212> PRT
   <213> Artificial Sequence
<220>
   <223> synthetically constructed MAB30 heavy chain amino acid sequence of variable domain
<400> 11
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Asp His
               20                  25                  30
   Tyr Met Asp Trp Val Arg Gln Ala Pro Gly Lys Gly Leu Glu Trp Val
           35                  40                  45
   Gly Arg Ile Arg Asn Lys Ala Ala Ile Tyr Thr Thr Glu Tyr Ala Ala
       50                  55                  60
   Ser Val Lys Gly Arg Phe Thr Ile Ser Arg Asp Asp Leu Lys Ser Ser
   65                  70                  75                  80
   Val Tyr Leu Gln Met Asn Ser Leu Lys Thr Asp Asp Thr Ala Ile Tyr
                   85                  90                  95
   Tyr Cys Ala Arg Ser Tyr Gly Tyr Phe Asp Tyr Trp Gly Gln Gly Thr
                   100                 105                 110
   Leu Val Thr Val Ser Ser
           115
<210> 12
   <211> 119
   <212> PRT
   <213> Artificial Sequence
<220>
   <223> synthetically constructed MAB42 heavy chain amino acid sequence of variable domain
<400> 12
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Ser Phe Asn Gly Tyr
               20                  25                  30
   Tyr Met His Trp Val Arg Gln Ala Pro Gly Gln Gly Leu Glu Trp Met
           35                  40                  45
   Gly Trp Ile Asn Leu Ser Ser Gly Gly Thr Asp Tyr Ala Gln Lys Phe
       50                  55                  60
   Gln Gly Trp Val Thr Leu Thr Arg Asp Thr Ser Ile Thr Thr Ala Tyr
   65                  70                  75                  80
   Met Glu Leu Ser Ser Leu Arg Ser Asn Asp Thr Ala Val Tyr Tyr Cys
                   85                  90                  95
   Ala Arg Ile Arg Pro Arg Thr Gly Gly Leu Asp Ser Trp Gly Gln Gly
               100                 105                 110
   Thr Leu Val Ile Val Ser Ser
           115
<210> 13
   <211> 121
   <212> PRT
   <213> Artificial Sequence
<220>
   <223> synthetically constructed MAB48 heavy chain amino acid sequence of variable domain
<400> 13
   Gln Val Gln Leu Val Gln Ser Gly Ala Glu Val Lys Lys Pro Gly Ser
    1               5                  10                  15
   Ser Val Lys Val Ser Cys Lys Ala Ser Gly Val Thr Phe Thr Ala Tyr
               20                  25                  30
   Ala Ile Ser Trp Val Arg Gln Ala Pro Gly Arg Gly Leu Glu Trp Met
           35                  40                  45
   Gly Gly Ile Ser Pro Leu Phe Gly Ile Val Asn Phe Gly Gln Asn Phe
       50                  55                  60
   Gln Gly Arg Val Thr Ile Thr Ala Asp Lys Ser Thr Gly Ala Ala Tyr
   65                  70                  75                  80
   Met Glu Leu Ser Ser Leu Ser Ser Glu Asp Thr Ala Met Tyr Tyr Cys
                   85                  90                  95
   Ala Arg Gly Pro Tyr Tyr Tyr Asp Arg Ser His Leu Asp Tyr Trp Gly
               100                 105                 110
   Gln Gly Thr Leu Val Thr Val Ser Ser
           115                 120
<210> 14
   <211> 121
   <212> PRT
   <213> Artificial Sequence
<220>
   <223> synthetically constructed MAB49 heavy chain amino acid sequence of variable domain
<400> 14
   Gln Val Gln Leu Val Gln Ser Gly Ala Glu Val Lys Arg Pro Gly Ser
    1               5                  10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Gly Met Phe Gly Thr Thr Asn Tyr Ala Gln Lys Phe
       50                  55                  60
   Gln Gly Arg Val Thr Ile Thr Ala Asp Glu Phe Thr Ser Thr Ala Tyr
   65                  70                  75                  80
   Met Glu Leu Thr Ser Leu Arg Ser Asp Asp Thr Ala Met Tyr Tyr Cys
                   85                  90                  95
   Ala Arg Asp Arg Asn Tyr Tyr Ala Ser Gly Thr Tyr Asp His Trp Gly
               100                 105                 110
   Gln Gly Thr Leu Val Thr Val Ser Ser
           115                 120
<210> 15
   <211> 120
   <212> PRT
   <213> Artificial Sequence
<220>
   <223> synthetically constructed MAB52 heavy chain amino acid sequence of variable domain
<400> 15
   Gln Val Leu Leu Val Gln Ser Gly Ala Glu Val Lys Lys Pro Gly Ser
   1 5 10 15
   Ser Val Asn Ile Ser Cys Lys Ala Ser Gly Gly Thr Phe Ser Asn Tyr
   20 25 30
   Ala Ile Ser Trp Val Arg Gln Ala Pro Gly Gln Gly Leu Asp Trp Met
   35 40 45
   Gly Arg Ile Ile Pro Ile Phe Gly Thr Ala Asn Tyr Ala Gln Lys Phe
   50 55 60
   Gln Gly Arg Leu Thr Ile Thr Ala Asp Glu Ser Thr Ser Thr Ala Tyr
   65 70 75 80
   Met Glu Leu Ser Ser Leu Arg Ser Glu Asp Thr Ala Val Phe Tyr Cys
   85 90 95
   Ala Ile Thr Lys Pro Gly Ser Val Tyr Ala Leu Asp Val Trp Gly Gln
   100 105 110
   Gly Thr Thr Val Thr Val Ser Ser
   115 120
<210> 16
   <211> 120
   <212> PRT
   <213> Artificial Sequence
<220>
   <223> synthetically constructed MAB53 heavy chain amino acid sequence of variable domain
<400> 16
   Gln Val Gln Leu Val Gln Ser Gly Ala Glu Val Arg Lys Pro Gly Ser
   1 5 10 15
   Ser Val Lys Val Ser Cys Lys Val Ser Gly Gly Ile Ile Arg Lys Tyr
   20 25 30
   Ala Ile Asn Trp Val Arg Gln Ala Pro Gly Gln Gly Leu Glu Trp Met
   35 40 45
   Gly Gly Ile Ile Ala Ile Phe Asn Thr Ala Asn Tyr Ala Gln Lys Phe
   50 55 60
   Gln Gly Arg Val Thr Ile Thr Ala Asp Glu Ser Thr Ser Thr Val Tyr
   65 70 75 80
   Met Glu Leu Ser Ser Leu Arg Ser Glu Asp Thr Ala Leu Tyr Tyr Cys
   85 90 95
   Ala Arg Gly Met Asn Tyr Tyr Ser Asp Tyr Phe Asp Tyr Trp Gly Gln
   100 105 110
   Gly Ser Leu Val Thr Val Ser Pro
   115 120
<210> 17
   <211> 120
   <212> PRT
   <213> Artificial Sequence
<220>
   <223> synthetically constructed MAB285 heavy chain amino acid sequence of variable domain
<400> 17
   Gln Val Gln Leu Val Gln Ser Gly Ala Glu Val Lys Lys Pro Gly Ala
    1               5                  10                  15
   Ser Val Lys Val Ser Cys Arg Ala Ser Gly Tyr Thr Phe Thr Gly Tyr
               20                  25                  30
   Tyr Met Gln Trp Val Arg Gln Ala Pro Gly Gln Gly Leu Glu Trp Met
           35                  40                  45
   Gly Phe Ile Asn Ala Asn Thr Gly Val Thr Asn Phe Ala Gln Lys Phe
       50                  55                  60
   Gln Gly Arg Val Thr Leu Thr Arg Asp Thr Ser Ile Ser Thr Ala Tyr
   65                  70                  75                  80
   Met Glu Leu Arg Arg Leu Thr Ser Ala Asp Thr Ala Val Tyr Tyr Cys
                   85                  90                  95
   Ala Arg Ala Pro Gln Trp Leu Ser Tyr Ser Phe Asp Ile Trp Gly Gln
               100                 105                 110
   Gly Thr Met Val Thr Val Ser Ser
           115                 120
<210> 18
   <211> 123
   <212> PRT
   <213> Artificial Sequence
<220>
   <223> synthetically constructed MAB321 heavy chain amino acid sequence of variable domain
<400> 18
   Glu Val Gln Leu Val Glu Ser Gly Ala Glu Val Arg Ser Pro Gly Ala
    1               5                  10                  15
   Ser Val Lys Leu Ser Cys Lys Ala Ser Ala Tyr Thr Phe Ile Asn Tyr
               20                  25                  30
   Tyr Leu His Trp Val Arg Gln Ala Pro Gly Gln Arg Leu Glu Trp Met
           35                  40                  45
   Gly Trp Ile Asn Pro Asp Ser Gly Val Thr Glu Tyr Ala Gln Thr Phe
       50                  55                  60
   Gln Gly Arg Val Thr Met Thr Arg Asp Thr Ser Ile Asn Thr Ala Tyr
   65                  70                  75                  80
   Leu Asp Leu Glu Arg Leu Thr Ser Asp Asp Thr Ala Val Tyr Tyr Cys
                   85                  90                  95
   Ala Arg Gly Phe Ile Pro Trp Gly Gly Lys Tyr Phe Tyr Leu Asp Tyr
               100                 105                 110
   Trp Gly Gln Gly Thr Leu Val Thr Val Ser Ser
           115                 120
<210> 19
   <211> 127
   <212> PRT
   <213> Artificial Sequence
<220>
   <223> synthetically constructed MAB322 heavy chain amino acid sequence of variable domain
<400> 19
   Gln Val Gln Leu Gln Gln Ser Gly Pro Gly Leu Val Lys Pro Ser Gln
    1               5                  10                  15
   Thr Leu Ser Leu Thr Cys Ser Val Ser Gly Ser Phe Ile Arg Ser Gly
               20                  25                  30
   Asp Tyr Asn Trp Ser Trp Ile Arg Gln Pro Pro Gly Lys Gly Leu Glu
           35                  40                  45
   Trp Ile Gly Tyr Ile Asp Asn Ser Gly Ser Thr His Tyr Asn Pro Ser
       50                  55                  60
   Leu Lys Ser Arg Val Ser Ile Ser Val Asp Thr Ser Lys Asn His Leu
   65                  70                  75                  80
   Ser Leu Lys Leu Ser Phe Val Thr Asp Ala Asp Thr Gly Val Tyr Tyr
                   85                  90                  95
   Cys Ala Gly Glu Gln Ala Ser Asp Ser Arg Gly Asn Tyr Tyr Tyr Tyr
               100                 105                 110
   Ala Met Asp Val Trp Gly Gln Gly Thr Pro Val Thr Val Ser Ser
           115                 120                 125
<210> 20
   <211> 120
   <212> PRT
   <213> Artificial Sequence
<220>
   <223> synthetically constructed MAB375 heavy chain amino acid sequence of variable domain
<400> 20
   Gln Val Gln Leu Gln Gln Ser Gly Pro Gly Leu Met Lys Pro Ser Glu
    1               5                  10                  15
   Thr Leu Ser Leu Ser Cys Thr Val Ser Gly Asp Ser Val Ser Ser Phe
               20                  25                  30
   Tyr Trp Ser Trp Ile Arg Gln Ser Pro Gly Lys Gly Leu Glu Trp Ile
           35                  40                  45
   Gly Tyr Leu Leu Tyr Ser Gly Asn Thr Lys Tyr Asn Pro Ser Leu Lys
       50                  55                  60
   Ser Arg Ala Thr Ile Ser Arg Asp Thr Ser Lys Asn Gln Leu Ser Leu
   65                  70                  75                  80
   Glu Leu Thr Ser Leu Thr Ala Ala Asp Thr Ala Val Tyr Tyr Cys Ala
                   85                  90                  95
   Arg Val Val Arg Trp Arg His Gly Gly Asp Leu Asp Val Trp Gly Gln
               100                 105                 110
   Gly Thr Met Val Thr Val Ser Ser
           115                 120
<210> 21
   <211> 126
   <212> **PRT**
   <213> Artificial Sequence
<220>
   <223> synthetically constructed MAB376 heavy chain amino acid sequence of variable domain
<400> 21
   Gln Val Gln Leu Val Gln Ser Gly Gly Asp Leu Val Gln Pro Gly Gly
    1               5                  10                  15
   Ser Leu Arg Leu Ser Cys Ala Val Ser Gly Phe Ile Phe Arg Lys Tyr
               20                  25                  30
   Ile Met Ser Trp Val Arg Gln Ala Pro Gly Lys Gly Pro Glu Trp Val
           35                  40                  45
   Ala Val Ile Ser Ser Ser Gly Asp Arg Thr Phe Tyr Ala Asp Ser Val
       50                  55                  60
   Glu Gly Arg Phe Ile Val Ser Arg Asp Asn Ser Lys Asp Thr Leu Phe
   65                  70                  75                  80
   Leu Gln Met Asn Ser Leu Arg Thr Glu Asp Thr Ala Met Tyr Tyr Cys
                   85                  90                  95
   Ala Lys Asp Leu Leu Gly Phe Cys Ser Gly Gly Asp Cys Leu Lys Val
               100                 105                 110
   Phe Asp Leu Trp Gly Arg Gly Thr Met Val Thr Val Ser Ser
           115                 120                 125
<210> 22
   <211> 120
   <212> PRT
   <213> Artificial Sequence
<220>
   <223> synthetically constructed MAB377 heavy chain amino acid sequence of variable domain
<400> 22
   Gln Val Gln Leu Leu Gln Ser Gly Pro Gly Leu Ile Lys Ala Ser Glu
    1               5                  10                  15
   Thr Leu Ser Leu Ser Cys Ser Val Ser Asn Asp Ser Val Ser Asn Tyr
               20                  25                  30
   Tyr Trp Ser Trp Ile Arg Gln Ser Pro Glu Lys Gly Leu Glu Trp Ile
           35                  40                  45
   Gly Tyr Leu Leu Tyr Ser Gly Asn Thr Lys Tyr Asn Pro Ser Leu Lys
       50                  55                  60
   Ser Arg Ala Ile Ile Ser Arg Asp Met Ser Lys Asn Gln Leu Ser Leu
   65                  70                  75                  80
   Arg Val Thr Ser Val Thr Ala Ala Asp Thr Ala Ile Tyr Tyr Cys Ala
                   85                  90                  95
   Arg Val Val Arg Trp Arg Phe Gly Gly Asp Met Asp Val Trp Gly Gln
               100                 105                 110
   Gly Thr Ala Val Thr Val Ser Thr
           115                 120
<210> 23
   <211> 120
   <212> PRT
   <213> Artificial Sequence
<220>
   <223> synthetically constructed MAB378 heavy chain amino acid sequence of variable domain
<400> 23
   Gln Val Gln Leu Gln Gln Ser Gly Pro Gly Leu Ile Lys Pro Ser Glu
    1               5                  10                  15
   Thr Leu Ser Leu Ser Cys Ser Val Ser Gly Asp Ser Val Asn Asn Tyr
               20                  25                  30
   Tyr Trp Ser Trp Ile Arg Gln Pro Pro Glu Lys Gly Leu Glu Trp Ile
           35                  40                  45
   Gly Tyr Leu Gln Tyr Ser Gly Ser Thr Lys Tyr Asn Pro Ser Leu Lys
       50                  55                  60
   Ser Arg Val Thr Ile Ser Arg Asp Thr Ser Lys Asn Gln Leu Ser Leu
   65                  70                  75                  80
   Lys Leu Thr Ser Val Thr Ala Ala Asp Thr Ala Ile Tyr Tyr Cys Ala
                   85                  90                  95
   Arg Val Val Arg Trp Arg His Gly Gly Asp Met Asp Val Trp Gly Gln
               100                 105                 110
   Gly Thr Ala Val Thr Val Ser Ser
           115                 120
<210> 24
   <211> 107
   <212> PRT
   <213> Homo sapiens
<220>
   <221> CHAIN
   <222> (1)...(107)
   <223> light chain amino acid sequence of constant kappa region
<400> 24
<210> 25
   <211> 106
   <212> PRT
   <213> Homo sapiens
<220>
   <221> CHAIN
   <222> (1)...(106)
   <223> light chain amino acid sequence of constant lambda region
<400> 25
   Gly Gln Pro Lys Ala Ala Pro Ser Val Thr Leu Phe Pro Pro Ser Ser
    1               5                  10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Ser Ser Pro
           35                  40                  45
   Val Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Arg Ser Tyr Ser Cys Gln Val Thr His Glu Gly Ser Thr Val
                   85                  90                  95
   Glu Lys Thr Val Val Pro Ala Glu Cys Ser
               100                 105
<210> 26
   <211> 214
   <212> PRT
   <213> Artificial Sequence
<220>
   <223> synthetically constructed MAB1 light chain amino acid sequence
<400> 26
   Asp Ile Gln Met Thr Gln Ser Pro Ser Ser Leu Ser Ala Ser Gly Gly
    1               5                  10                  15
   Asp Arg Val Thr Ile Thr Cys Arg Ala Ser Gln Ser Val Ser Thr Tyr
               20                  25                  30
   Leu Asn Trp Tyr Gln Gln Lys Pro Gly Lys Ala Pro Asn Leu Leu Val
           35                  40                  45
   Tyr Ala Val Ser Asn Leu Gln Arg Gly Val Pro Ser Arg Phe Ser Gly
       50                  55                  60
   Ser Gly Ser Gly Thr His Phe Thr Leu Thr Ile Ser Ser Leu Gln Pro
   65                  70                  75                  80
   Glu Asp Phe Ala Thr Tyr Tyr Cys Gln Gln Ser Tyr Ser Asp Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 27
   <211> 215
   <212> PRT
   <213> Artificial Sequence
<220>
   <223> synthetically constructed MAB8 light chain amino acid sequence
<400> 27
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Thr Ile Ser Lys Tyr
               20                  25                  30
   Leu Asn Trp Tyr Gln Gln Lys Pro Gly Arg Ala Pro Lys Leu Leu Ile
           35                  40                  45
   Tyr Ser Ala Ser Ser Leu Gln Ser Gly Val Pro Ser Arg Phe Thr Gly
       50                  55                  60
   Ser Gly Ser Gly Thr Asp Phe Thr Leu Thr Ile Thr Ser Leu Gln Pro
   65                  70                  75                  80
   Glu Asp Phe Ala Thr Tyr Tyr Cys Gln Gln Ser Tyr Arg Pro Ser Gln
                   85                  90                  95
   Ile Thr Phe Gly Pro Gly Thr Lys Val Asp Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 28
   <211> 214
   <212> PRT
   <213> Artificial Sequence
<220>
   <223> synthetically constructed MAB30 light chain amino acid sequence
<400> 28
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Asn Ala Pro Asn Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asp Thr Tyr Ser Pro
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 29
   <211> 216
   <212> **PRT**
   <213> Artificial Sequence
<220>
   <223> synthetically constructed MAB42 light chain amino acid sequence
<400> 29
   Gln Ser Ala Leu Thr Gln Pro Ala Ser Val Ser Gly Ser Ala Gly Gln
   1 5 10 15
   Ser Ile Thr Ile Ser Cys Thr Gly Thr Ser Ser Asp Val Gly Ala Tyr
   20 25 30
   Asn Phe Val Ser Trp Tyr Gln His His Pro Gly Lys Ala Pro Lys Leu
   35 40 45
   Met Ile Tyr Asp Val Asp Asn Arg Pro Ser Gly Val Ser Asn Arg Phe
   50 55 60
   Ser Gly Ser Lys Ser Gly Asp Thr Ala Ser Leu Thr Ile Ser Gly Leu
   65 70 75 80
   Gln Ala Glu Asp Glu Ala Asp Tyr Tyr Cys Ser Ser Tyr Arg Arg Asn
   85 90 95
   Gly Pro Trp Val Phe Gly Gly Gly Thr Lys Leu Thr Val Leu Gly Gln
   100 105 110
   Pro Lys Ala Ala Pro Thr Val Thr Leu Phe Pro Pro Ser Ser Glu Glu
   115 120 125
   Leu Gln Ala Asn Lys Ala Thr Leu Val Cys Leu Ile Ser Asp Phe Tyr
   130 135 140
   Pro Gly Ala Val Thr Val Ala Trp Lys Ala Asp Ser Ser Pro Val Lys
   145 150 155 160
   Ala Gly Val Glu Thr Thr Thr Pro Ser Lys Gln Ser Asn Asn Lys Tyr
   165 170 175
   Ala Ala Ser Ser Tyr Leu Ser Leu Thr Pro Glu Gln Trp Lys Ser His
   180 185 190
   Arg Ser Tyr Ser Cys Gln Val Thr His Glu Gly Ser Thr Val Glu Lys
   195 200 205
   Thr Val Val Pro Ala Glu Cys Ser
   210 215
<210> 30
   <211> 215
   <212> PRT
   <213> Artificial Sequence
<220>
   <223> synthetically constructed MAB48 light chain amino acid
<400> 30
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Gly Ser Ser
   20 25 30
   Asp Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Val Ser Ser Pro
   85 90 95
   Leu Thr Phe Gly Gly Gly Thr Lys Val Glu Ile Lys Arg Thr Val Ala
   100 105 110
   Ala Pro Ser Val Phe Ile Phe Pro Pro Ser Asp Glu Gln Leu Lys Ser
   115 120 125
   Gly Thr Ala Ser Val Val Cys Leu Leu Asn Asn Phe Tyr Pro Arg Glu
   130 135 140
   Ala Lys Val Gln Trp Lys Val Asp Asn Ala Leu Gln Ser Gly Asn Ser
   145 150 155 160
   Gln Glu Ser Val Thr Glu Gln Asp Ser Lys Asp Ser Thr Tyr Ser Leu
   165 170 175
   Ser Ser Thr Leu Thr Leu Ser Lys Ala Asp Tyr Glu Lys His Lys Val
   180 185 190
   Tyr Ala Cys Glu Val Thr His Gln Gly Leu Ser Ser Pro Val Thr Lys
   195 200 205
   Ser Phe Asn Arg Gly Glu Cys
   210 215
<210> 31
   <211> 214
   <212> PRT
   <213> Artificial Sequence
<220>
   <223> synthetically constructed MAB49 light chain amino acid
<400> 31
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Arg Tyr
   20 25 30
   Leu Asn Trp Tyr Gln Gln Lys Pro Gly Lys Ala Pro Lys Leu Leu Ile
   35 40 45
   Tyr Ser Ala Ser Ser Leu Gln Ser Gly Val Pro Ser Arg Phe Gly Gly
   50 55 60
   Ser Gly Ser Gly Thr Asp Phe Thr Leu Thr Ile Ser Ser Leu Gln Pro
   65 70 75 80
   Glu Asp Phe Ala Leu Tyr Tyr Cys Gln Gln Thr Tyr Ser Ile Pro Ile
   85 90 95
   Thr Phe Gly Gln Gly Thr Arg Leu Asp Phe Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 32
   <211> 215
   <212> PRT
   <213> Artificial Sequence
<220>
   <223> synthetically constructed MAB52 light chain amino acid
<400> 32
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Thr Ile Ser Thr Tyr
               20                  25                  30
   Leu Asn Trp Tyr Gln Gln Lys Pro Gly Lys Ala Pro Asn Leu Leu Ile
           35                  40                  45
   Tyr Thr Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Asp Ala Pro Thr
                   85                  90                  95
   Trp Thr Phe Gly Pro Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 33
   <211> 216
   <212> PRT
   <213> Artificial Sequence
<220>
   <223> synthetically constructed MAB53 light chain amino acid
<400> 33
   Glu Ile Val Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Arg Ser Asn
               20                  25                  30
   Asn Leu Ala Trp Tyr Gln His Lys Pro Gly Gln Ala Pro Arg Leu Leu
           35                  40                  45
   Ile Phe Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Ala Leu Thr Phe Gly Gly Gly Thr Lys Val Glu Ile Lys Arg Thr Val
               100                 105                 110
   Ala Ala Pro Ser Val Phe Ile Phe Pro Pro Ser Asp Glu Gln Leu Lys
           115                 120                 125
   Ser Gly Thr Ala Ser Val Val Cys Leu Leu Asn Asn Phe Tyr Pro Arg
       130                 135                 140
   Glu Ala Lys Val Gln Trp Lys Val Asp Asn Ala Leu Gln Ser Gly Asn
   145                 150                 155                 160
   Ser Gln Glu Ser Val Thr Glu Gln Asp Ser Lys Asp Ser Thr Tyr Ser
                   165                 170                 175
   Leu Ser Ser Thr Leu Thr Leu Ser Lys Ala Asp Tyr Glu Lys His Lys
               180                 185                 190
   Val Tyr Ala Cys Glu Val Thr His Gln Gly Leu Ser Ser Pro Val Thr
           195                 200                 205
   Lys Ser Phe Asn Arg Gly Glu Cys
       210                 215
<210> 34
   <211> 216
   <212> PRT
   <213> Artificial Sequence
<220>
   <223> synthetically constructed MAB285 light chain amino acid
<400> 34
   Gln Ser Val Leu Thr Gln Pro Pro Ser Ala Ser Gly Thr Pro Gly Gln
    1               5                  10                  15
   Arg Val Thr Ile Ser Cys Ser Gly Ser Ser Ser Asn Ile Gly Ser Asn
               20                  25                  30
   Pro Val Asn Trp Tyr Gln Gln Leu Pro Gly Thr Ala Pro Arg Leu Leu
           35                  40                  45
   Ile Tyr Ser Asn Asn Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Glu Asp Glu Ala Asp Tyr Tyr Cys Thr Ser Trp Asp Asp Ser Leu
                   85                  90                  95
   Asn Ala Trp Val Phe Gly Gly Gly Thr Arg Leu Thr Val Leu Gly Gln
               100                 105                 110
   Pro Lys Ala Ala Pro Ser Val Thr Leu Phe Pro Pro Ser Ser Glu Glu
           115                 120                 125
   Leu Gln Ala Asn Lys Ala Thr Leu Val Cys Leu Ile Ser Asp Phe Tyr
       130                 135                 140
   Pro Gly Ala Val Thr Val Ala Trp Lys Ala Asp Ser Ser Pro Val Lys
   145                 150                 155                 160
   Ala Gly Val Glu Thr Thr Thr Pro Ser Lys Gln Ser Asn Asn Lys Tyr
                   165                 170                 175
   Ala Ala Ser Ser Tyr Leu Ser Leu Thr Pro Glu Gln Trp Lys Ser His
               180                 185                 190
   Arg Ser Tyr Ser Cys Gln Val Thr His Glu Gly Ser Thr Val Glu Lys
           195                 200                 205
   Thr Val Val Pro Ala Glu Cys Ser
       210                 215
<210> 35
   <211> 213
   <212> PRT
   <213> Artificial Sequence
<220>
   <223> synthetically constructed MAB321 light chain amino acid
<400> 35
   Asp Ile Val Leu Thr Gln Ser Pro Pro Ser Leu Ser Ala Ser Val Gly
    1               5                  10                  15
   Asp Arg Val Thr Ile Thr Cys Arg Ala Ser Gln Ser Ile Asn Asn Tyr
               20                  25                  30
   Leu Asn Trp Tyr Gln Gln Lys Pro Gly Asn Ala Pro Arg Ile Leu Ile
           35                  40                  45
   Tyr Gly Ala Ser Ser Leu Val Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Arg Pro Leu Tyr
                   85                  90                  95
   Thr Phe Gly Pro Gly Thr Gln Leu Asp Val Lys Arg Thr Val Ala Ala
                   100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 36
   <211> 213
   <212> PRT
   <213> Artificial Sequence
<220>
   <223> synthetically constructed MAB322 light chain amino acid
<400> 36
   Asp Ile Val Met Thr Gln Ser Pro Ser Ser Leu Ser Ala Ser Val Gly
    1               5                  10                  15
   Asp Arg Val Thr Ile Thr Cys Arg Ala Ser Glu Ser Ile Ser Ala Tyr
               20                  25                  30
   Leu Asn Trp Tyr Gln His Thr Pro Gly Arg Ala Pro Lys Leu Leu Ile
           35                  40                  45
   Tyr Ala Ala Ser Ser Leu Glu Thr Gly Val Pro Ser Arg Phe Ser Gly
       50                  55                  60
   Ser Gly Ser Gly Thr Glu Phe Thr Leu Thr Ile Ser Gly Leu Gln Pro
   65                  70                  75                  80
   Glu Asp Phe Val Thr Tyr Tyr Cys Gln Gln Thr Tyr Asn Thr Pro Arg
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 37
   <211> 214
   <212> PRT
   <213> Artificial Sequence
<220>
   <223> synthetically constructed MAB375 light chain amino acid
<400> 37
   Asp Ile Gln Met Thr Gln Ser Pro Ser Phe Leu Ser Ala Ser Val Gly
    1               5                  10                  15
   Asp Arg Val Thr Phe Thr Cys Arg Ala Ser Gln Gly Ile Ala Ser Ser
               20                  25                  30
   Leu Ala Trp Tyr Gln Gln Lys Ala Gly Lys Ala Pro Lys Leu Leu Ile
           35                  40                  45
   Tyr Ala Ala Ser Thr Leu Glu Asp Gly Val Pro Ser Arg Phe Ser Gly
       50                  55                  60
   Ser Gly Phe Gly Thr Glu Phe Thr Leu Thr Ile Thr Ser Leu Gln Pro
   65                  70                  75                  80
   Glu Asp Phe Ala Thr Tyr Tyr Cys His Gln Val Asn Ser Tyr Pro Arg
                   85                  90                  95
   Thr Phe Gly Pro Gly Thr Thr Val Asp Ile Asn Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 38
   <211> 214
   <212> PRT
   <213> Artificial Sequence
<220>
   <223> synthetically constructed MAB376 light chain amino acid
<400> 38
   Asp Ile Gln Met Thr Gln Ser Pro Ser Thr Leu Ser Ala Ser Val Gly
    1               5                  10                  15
   Asp Thr Val Thr Ile Thr Cys Arg Ala Ser Gln Ser Ile Ser Thr Trp
               20                  25                  30
   Leu Ala Trp Phe Gln Gln Lys Pro Gly Arg Ala Pro Lys Leu Leu Ile
           35                  40                  45
   Tyr Gln Ala Ser Ser Leu Glu Gly Gly Val Pro Ser Arg Phe Ser Gly
       50                  55                  60
   Ser Gly Ser Gly Thr Asp Phe Asn Leu Thr Ile Ser Gly Leu Gln Pro
   65                  70                  75                  80
   Asp Asp Phe Ala Thr Tyr Tyr Cys Leu Gln Tyr Asn Thr Tyr Ser Lys
                   85                  90                  95
   Ser Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 39
   <211> 214
   <212> PRT
   <213> Artificial Sequence
<220>
   <223> synthetically constructed MAB377 light chain amino acid
<400> 39
   Asp Ile Gln Met Thr Gln Ser Pro Ser Phe Leu Ser Ala Ser Val Gly
    1               5                  10                  15
   Asp Arg Val Thr Ile Thr Cys Arg Ala Ser Gln Gly Ile Ala Thr Ser
               20                  25                  30
   Leu Ala Trp Tyr Gln Gln Lys Pro Gly Lys Ala Pro Arg Leu Leu Ile
           35                  40                  45
   Tyr Ala Ala Ser Thr Leu Glu Ser Gly Val Pro Ser Arg Phe Ser Gly
       50                  55                  60
   Gly Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Val Asn Ser Tyr Pro Arg
                   85                  90                  95
   Thr Phe Gly Pro Gly Thr Lys Leu Asp Val Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 40
   <211> 214
   <212> PRT
   <213> Artificial Sequence
<220>
   <223> synthetically constructed MAB378 light chain amino acid
<400> 40
   Asp Ile Gln Met Thr Gln Ser Pro Ser Phe Leu Ser Ala Ser Val Gly
    1               5                  10                  15
   Asp Arg Val Thr Met Thr Cys Arg Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Ile Tyr Tyr Cys Gln Gln Val Asn Gly Tyr Pro Arg
                   85                  90                  95
   Thr Phe Gly Pro Gly Thr Lys Val Asp Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 41
   <211> 1599
   <212> DNA
   <213> Homo sapiens
<220>
   <221> misc_feature
   <222> (1)...(1599)
   <223> IgG1 light chain nucleotide sequence of constant region
<220>
   <221> intron
   <222> (289)...(685)
<220>
   <221> intron
   <222> (731)...(848)
<220>
   <221> intron
   <222> (1208) ... (1277)
<400> 41
   gcctccacca agggcccatc agtcttcccc ctggcaccct ctaccaagag cacctctggg 60
   ggcacaacgg ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacatctgca acgtgaatca caagcccagc aacaccaagg tggacaagag agttggtgag 300
   aggccagcac agggagggag ggtgtctgct ggaagccagg ctcagcgctc ctgcctggac 360
   gcatcccggc tatgcagtcc cagtccaggg cagcaaggca ggccccgtct gcctcttcac 420
   ccggaggcct ctgcccgccc cactcatgct cagggagagg gtcttctggc tttttcccca 480
   ggctctgggc aggcacaggc taggtgcccc taacccaggc cctgcacaca aaggggcagg 540
   tgctgggctc agacctgcca agagccatat ccgggaggac cctgcccctg acctaagccc 600
   accccaaagg ccaaactctc cactccctca gctcggacac cttctctcct cccagattcc 660
   agtaactccc aatcttctct ctgcagagcc caaatcttgt gacaaaactc acacatgccc 720
   accgtgccca ggtaagccag cccaggcctc gccctccagc tcaaggcggg acaggtgccc 780
   tagagtagcc tgcatccagg gacaggcccc agccgggtgc tgacacgtcc acctccatct 840
   cttcctcagc acctgaactc ctggggggac cgtcagtctt cctcttcccc ccaaaaccca 900
   aggacaccct catgatctcc cggacccctg aggtcacatg cgtggtggtg gacgtgagcc 960
   acgaagaccc tgaggtcaag ttcaactggt acgtggacgg cgtggaggtg cataatgcca 1020
   agacaaagcc gcgggaggag cagtacaaca gcacgtaccg tgtggtcagc gtcctcaccg 1080
   tcctgcacca ggactggctg aatggcaagg agtacaagtg caaggtctcc aacaaagccc 1140
   tcccagcccc catcgagaaa accatctcca aagccaaagg tgggacccgt ggggtgcgag 1200
   ggccacatgg acagaggccg gctcggccca ccctctgccc tgagagtgac cgctgtacca 1260
   acctctgtcc ctacagggca gccccgagaa ccacaggtgt acaccctgcc cccatcccgg 1320
   gaggagatga ccaagaacca ggtcagcctg acctgcctgg tcaaaggctt ctatcccagc 1380
   gacatcgccg tggagtggga gagcaatggg cagccggaga acaactacaa gaccacgcct 1440
   cccgtgctgg actccgacgg ctccttcttc ctctatagca agctcaccgt ggacaagagc 1500
   aggtggcagc aggggaacgt cttctcatgc tccgtgatgc atgaggctct gcacaaccac 1560
   tacacgcaga agagcctctc cctgtccccg ggtaaatga                        1599
<210> 42
   <211> 363
   <212> DNA
   <213> Artificial Sequence
<220>
   <223> synthetically constructed MAB1 heavy chain variable domain nucleotide sequence
<400> 42
   caggtgcagc tgcaggagtc gggcccagga ctggtgaagc cttcggagac cctgtccctc 60
   atctgcagag tctctggtgg ctcgatcagt agtcattact ggagctggat ccggcagccc 120
   ccagggaagg gactggagtg gattggatat atttcttata gggggagaag caaccacaat 180
   ccttcccttg ggagacgagt ctctatgtca atagacacgt cggagaacca gttctccctg 240
   aacctgagct ctgtgatcgc tgcggacacg gccgtatatt actgtgcgag agatgctact 300
   gggatcagag aaatcaatgc tcttgatatc tggggccaag ggacaacggt caccgtctct 360
   tca                                                               363
<210> 43
   <211> 363
   <212> DNA
   <213> Artificial Sequence
<220>
   <223> synthetically constructed MAB8 heavy chain variable domain nucleotide sequence
<400> 43
   gaggtgcagc tggtggagtc tgggggaggc ctggtcaagc ctggggggtc cctgagactc 60
   tcctgtgcag cctctggttt cactttcagt acctatacta tgagttgggt ccgccaggct 120
   ccagggcagg ggctagagtg ggtctcgtcc attactagga ctagtagtaa tatatactac 180
   gcagactcag tggagggccg attcaccatc tccagagaca acgccaagaa ctcactgtat 240
   ctgcagatgc atagcctgag agtcgaagac acggctgtgt attactgtgc gagaatcagc 300
   ggggtagtgg gacctgtccc ctttgactac tggggccagg gaaccctgat caccgtctcc 360
   tct                                                               363
<210> 44
   <211> 354
   <212> DNA
   <213> Artificial Sequence
<220>
   <223> synthetically constructed MAB30 heavy chain variable domain nucleotide sequence
<400> 44
   gaggtgcagc tggtggagtc tgggggaggc ttggtccagc ctggagggtc cctgagactc 60
   tcctgtgcag cctctggatt caccttcagt gaccactaca tggactgggt ccgccaggct 120
   ccagggaagg ggctggagtg ggttggccgt attagaaata aagctgccat ttacaccaca 180
   gaatacgccg cgtctgtgaa aggcagattc accatctcaa gagatgattt aaagagctca 240
   gtgtatctgc aaatgaacag tctgaaaacc gacgacacgg ccatatatta ctgtgctagg 300
   agctatggat actttgacta ctggggccag ggaaccctgg tcaccgtctc ctca       354
<210> 45
   <211> 357
   <212> DNA
   <213> Artificial Sequence
<220>
   <223> synthetically constructed MAB42 heavy chain variable domain nucleotide sequence
<400> 45
   caggtgcagc tggtacagtc tggggctgag gtgaagaagc ctggggcctc agtgaaggtc 60
   tcctgcaagg cttctggata ttccttcaac ggctactata tgcactgggt gcgacaggcc 120
   cctggacaag ggcttgagtg gatgggttgg atcaacctga gcagtggtgg cacagattat 180
   gcacagaaat ttcaggggtg ggtcactttg accagggaca cgtccatcac cacagcctac 240
   atggagttga gcagcctgag atcgaacgac acggccgtgt attactgtgc gagaattaga 300
   cctcgcactg gtggacttga ctcctggggc cagggaaccc tggtcatcgt ctcctca    357
<210> 46
   <211> 363
   <212> DNA
   <213> Artificial Sequence
<220>
   <223> synthetically constructed MAB48 heavy chain variable domain nucleotide sequence
<400> 46
   caggtgcagc tggtgcagtc tggggctgag gtgaagaagc ctgggtcctc ggtgaaagtc 60
   tcctgcaagg cttctggagt caccttcacc gcctatgcta tcagttgggt gcgacaggcc 120
   cctggacgag ggcttgagtg gatgggaggg atcagccctt tgtttggaat agtaaatttc 180
   ggacagaact tccagggcag agtcacgatt accgcggaca aatccacggg cgcagcctac 240
   atggagctga gcagcctgag ctctgaggac acggccatgt attactgtgc gagaggaccc 300
   tattattacg atagaagtca cctagactac tggggccagg gaaccctggt caccgtctcc 360
   tca 363
<210> 47
   <211> 363
   <212> DNA
   <213> Artificial Sequence
<220>
   <223> synthetically constructed MAB49 heavy chain variable domain nucleotide sequence
<400> 47
   caggtgcagc tggtgcagtc tggggctgag gtgaagaggc ctgggtcctc ggtgaaggtc 60
   tcctgcaagg cttctggagg caccttcagc agttatgcta ttagctgggt gcgacaggcc 120
   cctggacaag ggcttgagtg gatgggaggg atcatcggta tgtttggaac aacaaactac 180
   gcacagaagt tccagggcag agtcacgatt accgcggacg aattcacgag cacagcctac 240
   atggagctga ccagcctgag atctgacgac acggccatgt attactgtgc gagagaccga 300
   aattactatg cttcggggac ttatgaccac tggggccagg gaaccctggt caccgtctcc 360
   tca 363
<210> 48
   <211> 360
   <212> DNA
   <213> Artificial Sequence
<220>
   <223> synthetically constructed MAB52 heavy chain variable domain nucleotide sequence
<400> 48
   caagtgctgc tggtgcagtc tggggctgaa gtgaagaagc ctgggtcctc ggtgaatatc 60
   tcttgcaagg cttctggagg cactttcagc aactatgcta tctcctgggt gcgacaggcc 120
   cctggacaag gtcttgactg gatgggaagg atcatcccta tctttggaac agcaaactac 180
   gcacagaaat tccagggcag actcaccatt accgcggacg aatccacgag cacagcctac 240
   atggaactga gcagcctgag atctgaagac acggccgtgt tttactgtgc gattactaaa 300
   ccggggtctg tctacgcttt ggacgtctgg ggccaaggga ccacggtcac cgtctcctca 360
<210> 49
   <211> 360
   <212> DNA
   <213> Artificial Sequence
<220>
   <223> synthetically constructed MAB53 heavy chain variable domain nucleotide sequence
<400> 49
   caggtgcagc tggtgcagtc tggggctgag gtgaggaagc cggggtcctc ggtgaaggtc 60
   tcctgcaagg tttctggagg catcattagg aaatatgcta tcaactgggt gcgacaggcc 120
   cccggacaag ggcttgagtg gatgggaggg atcatcgcta tctttaatac agcaaactat 180
   gcacagaaat tccagggcag agtcacgatt accgcggacg agtccacgag cacagtctac 240
   atggagctga gcagcctgag atctgaagac acggcccttt attactgtgc gagaggaatg 300
   aattactaca gtgactactt tgactactgg ggccagggaa gccttgtcac cgtctcccca 360
<210> 50
   <211> 360
   <212> DNA
   <213> Artificial Sequence
<220>
   <223> synthetically constructed MAB285 heavy chain variable domain nucleotide sequence
<400> 50
   caggtgcagc tggtgcagtc tggggctgag gtgaagaagc ctggggcctc agtgaaggtc 60
   tcctgccggg cttctggata caccttcacc ggctactata tgcagtgggt gcggcaggcc 120
   cctggccaag ggcttgagtg gatgggattc atcaatgcta acactggtgt cacaaacttt 180
   gctcagaagt ttcagggcag ggtcaccttg accagggaca cgtccatcag cacagcctac 240
   atggagctga ggaggctgac atctgccgac acggccgtgt attactgtgc gagagcgccc 300
   cagtggttat cgtattcttt tgatatctgg ggccaaggga caatggtcac cgtctcctca 360
<210> 51
   <211> 369
   <212> DNA
   <213> Artificial Sequence
<220>
   <223> synthetically constructed MAB321 heavy chain variable domain nucleotide sequence
<400> 51
   gaggtgcagc tggtggagtc tggggctgag gtgaggagcc ctggggcctc agtgaagctc 60
   tcctgcaagg cttctgcata caccttcatc aactactatc tgcactgggt gcgacaggcc 120
   cctggacaaa ggcttgagtg gatgggatgg atcaaccctg acagtggtgt cacagaatat 180
   gcacagacat ttcagggcag ggtcaccatg accagggaca cgtccatcaa tacagcctac 240
   ctggacctgg agagactgac atctgacgac acggccgtat attactgtgc gagaggtttt 300
   attccttggg gtgggaagta cttctacctt gactactggg gccagggaac cctggtcacc 360
   gtctcctca                                                         369
<210> 52
   <211> 381
   <212> DNA
   <213> Artificial Sequence
<220>
   <223> synthetically constructed MAB322 heavy chain variable domain nucleotide sequence
<400> 52
   caggtacagc tgcagcagtc agggccagga ctggtgaagc cttcacagac cctgtccctc 60
   acctgcagtg tatctggtag tttcatcaga agtggagatt ataattggag ttggatccgc 120
   cagcccccag ggaagggcct ggagtggatt gggtacatcg ataatagcgg gagcacccac 180
   tacaacccgt ccctcaagag tcgagttagc atatcagtgg acacgtccaa gaaccacttg 240
   tccctgaagc tgagttttgt gactgacgca gacacgggcg tgtattactg tgccggagaa 300
   caagcgtctg atagtcgtgg taattactac tactacgcta tggacgtctg gggccaaggg 360
   accccggtca ccgtctcctc a                                           381
<210> 53
   <211> 360
   <212> DNA
   <213> Artificial Sequence
<220>
   <223> synthetically constructed MAB375 heavy chain variable domain nucleotide sequence
<400> 53
   caggtgcagc tgcagcagtc gggccccgga ctgatgaagc cttcggagac cctgtccctc 60
   agctgcactg tctctggtga ctccgtcagt agtttttatt ggagttggat tcggcagtct 120
   ccaggaaagg gactggagtg gattgggtat ttgctttaca gtgggaatac caagtataat 180
   ccgtccctca agagtcgagc caccatatca agagacacgt ccaagaacca gttgtccctg 240
   gagttgacct ctctgaccgc tgcggacacg gccgtctact attgtgcgag agtggtgaga 300
   tggcgacatg gtggcgattt ggacgtctgg ggccaaggga ccacggtcac cgtctcctca 360
<210> 54
   <211> 378
   <212> DNA
   <213> Artificial Sequence
<220>
   <223> synthetically constructed MAB376 heavy chain variable domain nucleotide sequence
<400> 54
   caggtgcagc tggtgcagtc cgggggggac ttggtccagc cgggggggtc cctgagactg 60
   tcatgtgcag tctctggatt catctttaga aaatatatca tgagttgggt ccggcaggct 120
   ccagggaagg ggccggagtg ggtcgcagtt attagttcta gtggtgaccg gacattctac 180
   gccgactccg tggagggccg cttcatcgtc tccagagaca attccaagga cacactgttt 240
   ctgcaaatga acagcctgag aaccgaggac acggccatgt attactgtgc gaaagacctt 300
   ttgggatttt gtagtggtgg tgattgcctg aaggtcttcg atctctgggg ccgaggcacc 360
   atggtcactg tctcctca                                               378
<210> 55
   <211> 360
   <212> DNA
   <213> Artificial Sequence
<220>
   <223> synthetically constructed MAB377 heavy chain variable domain nucleotide sequence
<400> 55
   caggtgcagc tgctgcagtc gggcccagga ctgataaagg cttcggagac cctgtctctc 60
   agctgcagtg tctctaatga ctccgtcagt aattattatt ggagttggat ccggcagtcc 120
   ccagagaagg gactggagtg gattgggtat ttgctttata gtgggaatac caagtacaat 180
   ccctccctca agagtcgagc catcatatca agagacatgt ccaaaaatca gttgtccctc 240
   agagtgactt ctgtgaccgc tgcggacacg gccatatatt attgtgcgcg agtggtgaga 300
   tggcgatttg gtggtgatat ggacgtctgg ggtcaaggga ccgcggtcac cgtctccaca 360
<210> 56
   <211> 360
   <212> DNA
   <213> Artificial Sequence
<220>
   <223> synthetically constructed MAB378 heavy chain variable domain nucleotide sequence
<400> 56
   caggtgcagc tgcagcagtc gggcccagga ctgataaagc cttcggagac cctgtctctc 60
   agctgctctg tctctggtga ctccgtcaat aattattatt ggagttggat ccggcagccc 120
   ccagagaagg gactggagtg gattgggtat ctgcagtata gtgggagtac aaagtacaac 180
   ccctccctca agagtcgagt caccatatca agagacacgt ccaaaaacca gttgtccctg 240
   aagctgacct ctgtgaccgc tgcggacacg gccatatatt attgtgcgag agtggtgaga 300
   tggcgacatg gtggggatat ggacgtctgg ggccaaggga ccgcggtcac cgtctcctct 360
<210> 57
   <211> 324
   <212> DNA
   <213> Homo sapiens
<220>
   <221> misc_feature
   <222> (1)...(324)
   <223> light chain nucleotide sequence of constant kappa region
<400> 57
   cgaactgtgg ctgcaccatc tgtcttcatc ttcccgccat ctgatgagca gttgaaatct 60
   ggaactgcta gcgttgtgtg cctgctgaat aacttctatc ccagagaggc caaagtacag 120
   tggaaggtgg ataacgccct ccaatcgggt aactcccagg agagtgtcac agagcaggac 180
   agcaaggaca gcacctacag cctcagcagc accctgacgc tgagcaaagc agactacgag 240
   aaacacaaag tctacgcctg cgaagtcacc catcagggcc tgagctcgcc cgtcacaaag 300
   agcttcaaca ggggagagtg ttag                                        324
<210> 58
   <211> 318
   <212> DNA
   <213> Homo sapiens
<220>
   <221> misc_feature
   <222> (1)...(318)
   <223> light chain nucleotide sequence of constant lambda region
<400> 58
   ggtcagccca aggctgcccc ctctgtcact ctgttcccgc cctctagcga ggagcttcaa 60
   gccaacaagg ccacactggt gtgtctcata agtgacttct acccgggagc cgtgacagtg 120
   gcctggaagg cagatagcag ccccgtcaag gcgggagtgg agaccaccac accctccaaa 180
   caaagcaaca acaagtacgc ggccagcagc tatctgagcc tgacgcctga gcagtggaag 240
   tcccacagaa gctacagctg ccaggtcacg catgaaggga gcaccgtgga gaagacagtg 300
   gtccctgcag aatgctct                                               318
<210> 59
   <211> 321
   <212> DNA
   <213> Artificial Sequence
<220>
   <223> synthetically constructed MAB1 light chain variable domain nucleotide sequence
<400> 59
   gacatccaga tgacccagtc tccatcctcc ctgtctgcat ctggaggaga cagagtcacc 60
   atcacttgcc gggcaagtca gagtgttagt acgtatttaa attggtatca gcagaaacca 120
   gggaaagccc ctaacctcct ggtctatgct gtatccaatt tacaacgtgg cgtgccatca 180
   aggttcagtg gcagtggatc tgggacacat ttcactctca caatcagcag tctgcaacct 240
   gaggatttcg caacttacta ctgtcaacag agttacagtg accctctcac tttcggcgga 300
   gggaccaagg tggagatcaa a                                           321
<210> 60
   <211> 324
   <212> DNA
   <213> Artificial Sequence
<220>
   <223> synthetically constructed MAB8 light chain variable domain nucleotide sequence
<400> 60
   gacatccaga tgacccagtc tccatcttcc ctgtctgcat ctgtaggaga cagagtcacc 60
   atcacttgcc gggcaagtca gaccattagc aagtatttaa attggtatca gcagaagcca 120
   gggagagccc ctaaactcct gatctactct gcgtccagtt tgcaaagtgg ggtcccatca 180
   aggttcactg gcagtggatc tgggacagat ttcactctca ccatcaccag tctgcaacct 240
   gaagattttg caacttacta ctgtcaacag agttacagac cctcccagat cactttcggc 300
   cctgggacca aagtggatat caaa                                        324
<210> 61
   <211> 321
   <212> DNA
   <213> Artificial Sequence
<220>
   <223> synthetically constructed MAB30 light chain variable domain nucleotide sequence
<400> 61
   gacatccaga tgacccagtc tccttccacc ctgtctgcat ctgtaggaga cagagtcacc 60
   atcacttgcc gggccagtca gagtattagt agttggttgg cctggtatca gcagaaacca 120
   gggaacgccc ctaacctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tatgatactt attctccgac gttcggccaa 300
   gggaccaagg tggaaatcaa a                                           321
<210> 62
   <211> 330
   <212> DNA
   <213> Artificial Sequence
<220>
   <223> synthetically constructed MAB42 light chain variable domain nucleotide sequence
<400> 62
   cagtctgccc tgactcagcc tgcctccggg tctgggtctg ctggacaggc gatcaccatc 60
   tcctgcactg gaaccggcac tgacgtctgt gcttataact ttgtctcctg gtaccaacac 120
   caccccggcg aagcccccaa actcatgatt tatgatgtcg ataatcggcc ctcatgggtt 180
   tctaatcgct tctctggctc caagtctggt aacacggcct ccctgaccat ctctgggctc 240
   caggctgagg acgaggctga ttactactgc agctcatata gaaggaacgg cccttgcttg 300
   ttcggcggag ggaccaagct gaccgtcctg                                  330
<210> 63
   <211> 324
   <212> DNA
   <213> Artificial Sequence
<220>
   <223> synthetically constructed MAB48 light chain variable domain nucleotide sequence
<400> 63
   gaaattgtgt tgacgcagtc tccaggcacc ctgtctttgt ctccagggga aagagccacc 60
   ctctcctgca gggccagtca gagtgttggc agcagcgact tagcctggta ccagcagaaa 120
   cctggccagg ctcccaggct cctcatatat ggtgcatcca gccgggccac tggcatccca 180
   gacaggttca gtggcagtgg gtctgggaca gacttcactc tcaccatcag cagactggag 240
   cctgaagatt ttgcagtgta ttactgtcag cagtatgtca gttcacccct cactttcggc 300
   ggagggacca aggtggagat caag                                        324
<210> 64
   <211> 321
   <212> DNA
   <213> Artificial Sequence
<220>
   <223> synthetically constructed MAB49 light chain variable domain nucleotide sequence
<400> 64
   gacatccaga tgacccagtc tccatcctcc ctgtctgcat ctgtaggaga cagagtcacc 60
   atcacttgcc gggcaagtca gagcattagc aggtatttaa attggtatca gcagaaacca 120
   gggaaagccc ctaaactcct gatctattct gcatccagtt tgcaaagtgg ggtcccatca 180
   aggttcggtg gcagtggatc tgggacagat ttcactctca ccatcagcag tctgcaacct 240
   gaagattttg cactttacta ctgtcaacag acttacagta tcccgatcac cttcggccaa 300
   gggacacgac tggactttaa a                                           321
<210> 65
   <211> 324
   <212> DNA
   <213> Artificial Sequence
<220>
   <223> synthetically constructed MAB52 light chain variable domain nucleotide sequence
<400> 65
   gacatccaga tgacccagtc tccatcctcc ctgtctgcat ctgtaggaga cagagtcact 60
   atcacttgcc gggcaagtca gaccattagc acctatttaa attggtatca gcagaaacca 120
   gggaaagccc ctaacctcct gatctatact gcatccagtt tgcaaagcgg ggtcccatca 180
   agattcagtg gcagtggatc tgggacagat ttcactctca ccatcagcag tctgcaacct 240
   gaagattttg caacttatta ctgtcaacag agttacgatg cccccacgtg gaccttcggc 300
   ccagggacca aggtggaaat caaa                                        324
<210> 66
   <211> 327
   <212> DNA
   <213> Artificial Sequence
<220>
   <223> synthetically constructed MAB53 light chain variable domain nucleotide sequence
<400> 66
   gaaattgtgt tgacacagtc tccaggcacc ctgtctttgt ctccagggga aagagccacc 60
   ctctcctgca gggccagtca gagtgttaga agcaacaact tagcctggta ccagcacaaa 120
   cctggccagg ctcccaggct cctcatcttt ggtgcatcca gcagggccac tggcatccca 180
   gacaggttca gtggcagtgg gtctgggaca gacttcactc tcaccatcag cagactggag 240
   cctgaagatt ttgcagtata ttactgtcag cagtatggta gctcacctgc gctcactttc 300
   ggcggaggga ccaaggtgga gatcaaa                                     327
<210> 67
   <211> 330
   <212> DNA
   <213> Artificial Sequence
<220>
   <223> synthetically constructed MAB285 light chain variable domain nucleotide sequence
<400> 67
   cagtctgtgc tgactcagcc accctcagcg tctgggaccc ccgggcagag ggtcaccatc 60
   tcttgttctg gaagcagctc caacatcgga agtaatcctg taaactggta ccagcagctc 120
   ccaggaacgg cccccagact tctcatctat agtaataatc agcggccctc aggggtccct 180
   gaccgattct ctggctccaa gtctggcacc tcagcctccc tggccatcag tgggctccgg 240
   tccgaggatg aggctgatta ctactgtaca tcatgggatg acagcctgaa tgcttgggtg 300
   ttcggcgggg ggaccaggct gaccgtccta                                  330
<210> 68
   <211> 321
   <212> DNA
   <213> Artificial Sequence
<220>
   <223> synthetically constructed MAB321 light chain variable domain nucleotide sequence
<400> 68
   gatatcgtgt tgactcagtc tccaccctcc ctgtctgcat ctgtggggga cagagtcacc 60
   atcacttgcc gggcaagtca gagcattaat aactacttaa attggtatca acagaaacca 120
   gggaacgccc caagaatact aatctatggt gcatccagtt tggtaagtgg ggtcccatca 180
   aggttcagtg gcagtggatc tgggacagat ttcaccctca ccatcagcag tctgcaacct 240
   gaagattttg caacttacta ctgtcaacag agttaccggc ccctgtacac ttttggcccg 300
   gggacccagc tggatgtcaa a                                           321
<210> 69
   <211> 321
   <212> DNA
   <213> Artificial Sequence
<220>
   <223> synthetically constructed MAB322 light chain variable domain nucleotide sequence
<400> 69
   gatatcgtga tgacccagtc tccatcttcc ctgtctgcat ctgtgggaga cagagtcacc 60
   atcacttgcc gggcaagtga gagcattagc gcttatttaa attggtatca gcacacacca 120
   gggagagccc ctaagctcct gatctatgct gcctccagtt tggaaactgg ggtcccatca 180
   aggttcagtg gcagtggatc tggcacagaa ttcactctca ccatcagcgg tctgcaacct 240
   gaagattttg tcacttacta ctgtcaacag acttacaata cccctcggac cttcggccaa 300
   gggaccaagg tggaaatcaa a                                           321
<210> 70
   <211> 321
   <212> DNA
   <213> Artificial Sequence
<220>
   <223> synthetically constructed MAB375 light chain variable domain nucleotide sequence
<400> 70
   gatatccaga tgacccagtc tccatccttc ttgtctgcat ctgtgggaga cagagtcacc 60
   ttcacttgcc gggccagtca gggcattgcc agttctttag cctggtatca gcaaaaagca 120
   gggaaagccc ctaagctcct gatctatgct gcttctactt tggaagatgg ggtcccatca 180
   aggttcagcg gcagtggatt tgggacagaa ttcactctca caatcaccag cctgcagcct 240
   gaagattttg caacctatta ctgtcatcag gtgaatagtt accctcggac tttcggccct 300
   gggaccacag tggatatcaa c                                           321
<210> 71
   <211> 322
   <212> DNA
   <213> Artificial Sequence
<220>
   <223> synthetically constructed MAB376 light chain variable domain nucleotide sequence
<400> 71
   gatatccaga tgacccagtc tccttccacc ctgtctgcat ctgtgggaga cacagtcacc 60
   atcacttgcc gggccagtca gagtattagt acttggttgg cctggtttca gcagaaacca 120
   gggagagccc ctaaactcct gatctatcag gcgtctagtt tggaaggtgg ggtcccatca 180
   aggttcagcg gcagtgggtc tgggacagac ttcaacctca ccatcagcgg cctgcagcct 240
   gatgattttg caacttatta ctgcctacaa tataacactt attcgaagtc attcggccaa 300
   gggaccaagg tggaaatcaa ac                                          322
<210> 72
   <211> 322
   <212> DNA
   <213> Artificial Sequence
<220>
   <223> synthetically constructed MAB377 light chain variable domain nucleotide sequence
<400> 72
   gatatccaga tgacccagtc tccatccttc ttgtctgcat ctgtcggaga cagagtcacc 60
   atcacctgcc gggccagtca gggcattgcc acttctttag cctggtatca gcaaaaacct 120
   gggaaagccc cgaggctcct gatctatgct gcatccactt tggaaagtgg ggtcccatca 180
   aggttcagcg gcggtggatc tgggacagac ttcactctca caatcagcag tctgcagccc 240
   gaagattttg ctgtttatta ctgtcaacag gttaactcct atcctcggac tttcggccct 300
   gggaccaaac tggatgtcaa ac                                          322
<210> 73
   <211> 322
   <212> DNA
   <213> Artificial Sequence
<220>
   <223> synthetically constructed MAB378 light chain variable domain nucleotide sequence
<400> 73
   gatatccaga tgacccagtc tccatccttc ttgtctgcat ctgtaggaga cagagtcacc 60
   atgacctgcc gggccagtca gggcattagc agttatttag cctggtatca gcaaaaacca 120
   gggaaagccc ctaagctcct gatctatgct gcatcgactt tggaaagtgg ggtcccatca 180
   aggttcagcg gcagtggatc tgggacagaa ttcactctca caatcagcag cctgcagccc 240
   gaagattttg caatttatta ctgtcaacag gttaatggtt accctcggac tttcggccct 300
   gggaccaaag tggatatcaa ac                                          322
<210> 74
   <211> 15
   <212> PRT
   <213> Artificial Sequence
<220>
   <223> synthetically constructed peptide derived from HA2
<400> 74
   Arg Gly Leu Phe Gly Ala Ile Ala Gly Phe Ile Glu Asn Gly Trp
    1               5                  10                  15
<210> 75
   <211> 450
   <212> PRT
   <213> Artificial Sequence
<220>
   <223> synthetically constructed MAB53 heavy chain amino acid sequence
<400> 75
   Gln Val Gln Leu Val Gln Ser Gly Ala Glu Val Arg Lys Pro Gly Ser
   1 5 10 15
   Ser Val Lys Val Ser Cys Lys Val Ser Gly Gly Ile Ile Arg Lys Tyr
   20 25 30
   Ala Ile Asn Trp Val Arg Gln Ala Pro Gly Gln Gly Leu Glu Trp Met
   35 40 45
   Gly Gly Ile Ile Ala Ile Phe Asn Thr Ala Asn Tyr Ala Gln Lys Phe
   50 55 60
   Gln Gly Arg Val Thr Ile Thr Ala Asp Glu Ser Thr Ser Thr Val Tyr
   65 70 75 80
   Met Glu Leu Ser Ser Leu Arg Ser Glu Asp Thr Ala Leu Tyr Tyr Cys
   85 90 95
   Ala Arg Gly Met Asn Tyr Tyr Ser Asp Tyr Phe Asp Tyr Trp Gly Gln
   100 105 110
   Gly Ser Leu Val Thr Val Ser Pro Ala Ser Thr Lys Gly Pro Ser Val
   115 120 125
   Phe Pro Leu Val Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 76
   <211> 216
   <212> PRT
   <213> Artificial Sequence
<220>
   <223> synthetically constructed MAB53 light chain amino acid sequence
<400> 76
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Arg Ser Asn
               20                  25                  30
   Asn Leu Ala Trp Tyr Gln His Lys Pro Gly Gln Ala Pro Arg Leu Leu
           35                  40                  45
   Ile Phe Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Ala Leu Thr Phe Gly Gly Gly Thr Lys Val Glu Ile Lys Arg Thr Val
               100                 105                 110
   Ala Ala Pro Ser Val Phe Ile Phe Pro Pro Ser Asp Glu Gln Leu Lys
           115                 120                 125
   Ser Gly Thr Ala Ser Val Val Cys Leu Leu Asn Asn Phe Tyr Pro Arg
       130                 135                 140
   Glu Ala Lys Val Gln Trp Lys Val Asp Asn Ala Leu Gln Ser Gly Asn
   145                 150                 155                 160
   Ser Gln Glu Ser Val Thr Glu Gln Asp Ser Lys Asp Ser Thr Tyr Ser
                   165                 170                 175
   Leu Ser Ser Thr Leu Thr Leu Ser Lys Ala Asp Tyr Glu Lys His Lys
               180                 185                 190
   Val Tyr Ala Cys Glu Val Thr His Gln Gly Leu Ser Ser Pro Val Thr
           195                 200                 205
   Lys Ser Phe Asn Arg Gly Glu Cys
       210                 215
<210> 77
   <211> 10
   <212> PRT
   <213> Artificial Sequence
<220>
   <223> synthetically constructed CDR1 region of IGHV1-69 01 heavy chain
<400> 77
   Gly Gly Ile Ile Arg Lys Tyr Ala Ile Asn
   1               5                   10
<210> 78
   <211> 18
   <212> PRT
   <213> Artificial Sequence
<220>
   <223> synthetically constructed CDR2 region of IGHV1-69 01 heavy chain
<400> 78
   Gly Gly Ile Ile Ala Ile Phe Asn Thr Ala Asn Tyr Ala Gln Lys Phe
   1               5                   10                  15
   Gln Gly
<210> 79
   <211> 13
   <212> PRT
   <213> Artificial Sequence
<220>
   <223> synthetically constructed CDR3 region of IGHV1-69 01 heavy chain
<400> 79
   Ala Arg Gly Met Asn Tyr Tyr Ser Asp Tyr Phe Asp Tyr
   1               5                   10
<210> 80
   <211> 12
   <212> PRT
   <213> Artificial Sequence
<220>
   <223> synthetically constructed CDR1 region of IGKV3-20 01 light chain
<400> 80
   Arg Ala Ser Gln Ser Val Arg Ser Asn Asn Leu Ala
   1               5                   10
<210> 81
   <211> 7
   <212> PRT
   <213> Artificial Sequence
<220>
   <223> synthetically constructed CDR2 region of IGKV3-20 01 light chain
<400> 81
   Gly Ala Ser Ser Arg Ala Thr
   1               5
<210> 82
   <211> 10
   <212> PRT
   <213> Artificial Sequence
<220>
   <223> synthetically constructed CDR3 region of IGKV-20 01 light chain
<400> 82
   Gln Gln Tyr Gly Ser Ser Pro Ala Leu Thr
    1               5                  10
<210> 83
   <211> 120
   <212> PRT
   <213> Artificial Sequence
<220>
   <223> synthetically constructed MAB53 heavy chain variable region IGHV1-69 01
<400> 83
   Gln Val Gln Leu Val Gln Ser Gly Ala Glu Val Arg Lys Pro Gly Ser
    1               5                  10                  15
   Ser Val Lys Val Ser Cys Lys Val Ser Gly Gly Ile Ile Arg Lys Tyr
               20                  25                  30
   Ala Ile Asn Trp Val Arg Gln Ala Pro Gly Gln Gly Leu Glu Trp Met
           35                  40                  45
   Gly Gly Ile Ile Ala Ile Phe Asn Thr Ala Asn Tyr Ala Gln Lys Phe
       50                  55                  60
   Gln Gly Arg Val Thr Ile Thr Ala Asp Glu Ser Thr Ser Thr Val Tyr
   65                  70                  75                  80
   Met Glu Leu Ser Ser Leu Arg Ser Glu Asp Thr Ala Leu Tyr Tyr Cys
                   85                  90                  95
   Ala Arg Gly Met Asn Tyr Tyr Ser Asp Tyr Phe Asp Tyr Trp Gly Gln
               100                 105                 110
   Gly Ser Leu Val Thr Val Ser Pro
           115                 120
<210> 84
   <211> 109
   <212> PRT
   <213> Artificial Sequence
<220>
   <223> synthetically constructed MAB53 light chain variable region IGKV3-20 01
<400> 84
   Glu Ile Val Leu Thr Gln Ser Pro Gly Thr Leu Ser Leu Ser Pro Gly
    1               5                  10                  15
   Glu Arg Ala Thr Leu Ser Cys Arg Ala Ser Gln Ser Val Arg Ser Asn
               20                  25                  30
   Asn Leu Ala Trp Tyr Gln His Lys Pro Gly Gln Ala Pro Arg Leu Leu
           35                  40                  45
   Ile Phe Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Ala Leu Thr Phe Gly Gly Gly Thr Lys Val Glu Ile Lys
               100                 105

## Description

### Technical Field

The invention relates to the field of passive immunization against influenza. More particularly, antibodies that bind near to the HA₀ maturation cleavage site consensus sequence of influenza hemagglutinin A, including antibodies secreted by human cells.

### Background Art

The hemagglutinin protein of influenza virus has a globular head domain which is highly heterogeneous among flu strains and a stalk region containing a fusion site which is needed for entry into the cells. The hemagglutinin protein (HA₀) is activated to permit the fusion site to effect virulence by cleavage into HA₁ and HA₂ portions which remain coupled using disulfide bonds but undergo a conformational change. This cleavage site contains a consensus sequence which is shared both by influenza A and influenza B and by the various strains of influenza A and B.

Bianchi, E., et al., J. Virol. (2005) 79:7380-7388 describe a "universal" influenza B vaccine based on the consensus sequence of this cleavage site which was able to raise antibodies in mice when conjugated to the outer membrane protein complex of *Neisseria meningitidis.* Monoclonal antibodies which appear to bind to the consensus sequence were also described. In addition, successful passive transfer of antiserum was observed in mice. Prior vaccines, such as those described in WO2004/080403 comprising peptides derived from the M2 and/or HA proteins of influenza are subject to inducing antibodies that are either of weak efficacy or are not effective across.

Prabhu et al (J.Vir. vol. 83, no. 6, pp. 2553-2562) and WO2010074656 report on antibody 1C9 which is specific to the antigenically conserved fusion peptide of HA2 and can contribute to the cross-clade protection of mice infected with H5N1 virus.

### Disclosure of the Invention

The invention provides a human monoclonal antibody, optionally a bispecific antibody, or immunoreactive fragment thereof, comprising a heavy chain comprising CDR1 of the sequence GGIIRKYAIN (SEQ ID NO:77), CDR2 of the sequence GGIIAIFNTANYAQKFQG (SEQ ID NO:78), and CDR3 of the sequence ARGMNYYSDYFDY (SEQ ID NO:79).and a light chain comprising CDR1 of the sequence RASQSVRSNNLA (SEQ ID NO:80), a CDR2 of the sequence GASSRAT (SEQ ID NO:81), and a CDR3 of the sequence QQYGSSPALT (SEQ ID NO:82), wherein the antibody or fragment binds to HA₀ and HA₂ protein stalk region from influenza viral clades that include at least one clade from each of Group 1 and Group 2 of influenza A. Such antibodies are able to confer passive immunity in the event of a pandemic caused, for example, by a previously unidentified influenza strain or a strain against which protection is not conferred by the seasonal vaccines currently available. Since the antibodies bind across many strains, indicative of targeting an essential site and thus likely to be included even in previously unencountered strain, such a vaccine would be effective in such circumstances. Such antibodies are also useful to ameliorate or prevent infection in subjects for whom vaccination failed to produce a fully protective response or who are at high risk due to a weak immune system (e.g., the very young, the elderly, transplant patients, cancer or HIV chemotherapy treated patients).

Thus, the invention is directed to monoclonal antibodies or immunoreactive fragments thereof that are broadly crossreactive with influenza A virus of Group 1 including H1, H2, H5, H6, H8, H9, H11, H13, H16 or Group 2 including H3 and H7 as type specimens, or that show cross-Group reactivity. The antibodies bind specifically to an epitope contained in the HA₀ protein of the influenza virus and recognize the native trimeric form of HA. As is well understood in the art, non-immunoglobulin based proteins may have similar epitope recognition properties as an antibody and can also provide suitable embodiments, including binding agents based on fibronectin, transferrin, lipocalin, or nucleic acid based aptamers.

In other aspects, the invention is directed to methods to use the antibodies and fragments of the invention for passively inhibiting viral infection in subjects. The invention is also directed to recombinant materials and methods to produce these antibodies or fragments.

### Brief Description of the Drawings

Figures 1A and 1B show the results of binding by MAB53 and MAB8 with respect to HA₀ protein from various influenza clades tested by ELISA. Figure 1C shows that MAB53 binds to native trimer, expressed in HEK293 cells.
Figures 2A and 2B show the results of binding of MAB53 and MAB8 *versus* HA₀ protein from various clades as tested by FortéBio® biosensor.
Figure 3A shows the extent of binding as tested by ELISA of MAB53 with respect to HA₀ as an intact protein and the cleavage fragment HA₁. Figure 3B shows the extent of binding of MAB53 to a peptide denoted CP from HA₂.
Figures 4A and 4B show the results of a FortéBio® assay demonstrating that MAB53 competes with MAB8, but not with MAB30.
Figures 5A and 5B show CDR mapping according to Kabat number of MAB53 heavy and light chain variable regions. IGHV1-69*01 is SEQ ID NO:83 and IGKV3-20*01 is SEQ ID NO:84.
Figure 6 shows neutralization of H1N1 by various amounts of MAB53, as measured by in vitro plaque assay.
Figures 7A and 7B show survival times for mice challenged with H1N1 (panel A) or H5N1 (panel B) as a function of administration of various amounts of MAB53.
Figure 8 shows the effect of post-infection treatment of H5N1 with MAB53.

### Modes of Carrying Out the Invention

The present invention provides useful antibodies including providing effective means to identify cells that secrete such antibodies so that the relevant coding sequences can be retrieved and stored for subsequent and facile recombinant production of such antibodies. The method includes a binary logic based design of a screening procedure.

Such a procedure can readily be applied to human cells using, in particular, the CellSpot™ method described in U.S. patent 7,413,868. Briefly, the method is able to screen individual cells obtained from human (or other) subjects in high throughput assays taking advantage of labeling with particulate labels and microscopic observation. In one illustrative embodiment, even a single cell can be analyzed for antibodies it secretes by allowing the secreted antibodies to be adsorbed on, or coupled to, a surface and then treating the surface with desired antigens each coupled to a distinctive particulate label. The footprint of a cell can therefore be identified with the aid of a microscope. Using this technique, millions of cells can be screened for desirable antibody secretions and even rare antibodies, such as those herein desirable for passive influenza immunization across strains can be recovered. Since human subjects have existing antibodies to at least some influenza strains, and since the antibodies obtained by the method of the invention bind a conserved sequence, these antibodies serve the purpose of addressing new strains as well as strains with which human populations have experience.

The disclosure provides a method to identify a monoclonal antibody of the present invention that binds to a location near the hemagglutinin (HA₀) cleavage site consensus sequence. The method comprises contacting candidate monoclonal antibodies or fragments with: i) a peptide consisting essentially of an amino acid sequence upstream of or downstream of said consensus sequence, but lacking said consensus sequence; ii) a peptide consisting essentially of an amino acid sequence upstream of said consensus sequence and including said consensus sequence; and iii) a peptide consisting essentially of an amino acid sequence downstream of said consensus sequence and including said consensus sequence; wherein a monoclonal antibody that binds to the peptide of ii) and iii) but not to the peptide of i) is identified as a peptide that binds specifically to the HA₀ cleavage site consensus sequence. Other combinations could also be used, as will be evident to the skilled artisan, as long as binary logic is followed. For example, i) could be a peptide consisting essentially of an amino acid upstream of the consensus sequence of a first strain and lacking the consensus sequence , with ii) being the whole HA₀ sequence from the first strain and iii) being the whole HA₀ sequence from a second strain. Shorter portions could also be used. For further confirmation, an isolated peptide from the conserved region can also be used, although the information derived from the larger protein domains is believed to be more informative regarding recognition of the intact antigen.

This method is not limited to employing the CellSpot™ technique, nor is it limited to human antibodies. The binary logic of this method can be employed in any alternative screening method. Likewise, it can be applied to other diversity libraries besides natural immunoglobulins.

The method relies on binary logic wherein peptides that contain the desired consensus sequence and additional upstream and/or downstream portions are used as test peptides and their ability to complex antibodies as compared to regions lacking the consensus sequence is assessed. Thus, patterns are obtained whereby cells secreting the appropriate antibodies can be instantly identified.

In one illustrative embodiment, three antigens are used to assess the secreted antibody population. The first peptide is all or substantially all of the amino acid sequence upstream of the consensus sequence contained in HA₀ and is coupled to a particulate label of, say, red. A second test antigen contains these upstream sequences, but contains also the consensus sequence and is labeled with particle of a different color, for example, blue. A third test peptide contains the consensus sequence and all or substantially all of the downstream regions of the HA₀ protein and is labeled with a third color particulate, for example, green. (By upstream portion is meant toward the N-terminus from the consensus sequence and by downstream portion the continuation of the amino acid sequence from the consensus sequence toward the C-terminus. By "substantially all" is meant lacking only one or a few non-essential amino acids.) Antibodies that bind to the consensus sequence will bind both the green and blue particulate labeled peptides but will not bind the red labeled upstream sequence lacking the consensus sequence. If desired, the specificity can be confirmed by adding a fourth peptide representing only the downstream portion without the consensus sequence bound, for example, to a yellow particulate label, wherein the yellow particulate label will not be bound to the antibody. Of course, it does not matter whether the upstream or downstream portion is chosen as the negative control.

The cleavage site for various strains of influenza A and influenza B is known. For example, the above cited article by Bianchi, *et al.,* shows in Table 1 the sequence around the cleavage site of several such strains:

**Table 1 Consensus sequence of the solvent-exposed region of the influenza A and B virus maturational cleavage sites**

| Virus/subtype | Strain | | Sequence*^{a}* | |
|---|---|---|---|---|
| A/H3/HA₀ | Consensus | NVPEKQTR | ↓ | GIFGAIAGFIE |
| | | (SEQ ID NO:1) | | (SEQ ID NO: 2) |
| A/H1/HA₀ | Consensus | NIPSIQSR | ↓ | GLFGAIAGFIE |
| | | (SEQ ID NO:3) | | (SEQ ID NO: 4) |
| B/HA₀ | *_{b}* Consensus | PAKLLKER | ↓ | GFFGAIAGFLE |
| | | (SEQ ID NO:5) | | (SEQ ID NO: 6) |

| | | | | |
|---|---|---|---|---|
| *^{a}*The position of cleavage between HA₁ and HA₂ is indicated by the arrow. *^{b}*The consensus is the same for both the Victoria and Yamagata lineages. | | | | |

As indicated, strict consensus occurs starting with the arginine residue upstream of the cleavage site and thus preferred consensus sequences included in the test peptides of the invention have the sequence RGI/L/F FGAIAGFLE (SEQ ID NO:7). It may be possible to use only a portion of this sequence in the test peptides.

Once cells that secrete the desired antibodies have been identified, it is straightforward to retrieve the nucleotide sequences encoding them and to produce the desired antibodies on a large scale recombinantly. This also enables manipulation of the antibodies so that they can be produced, for example, as single-chain antibodies or in terms of their variable regions only.

The retrieved nucleic acids may be physically stored and recovered for later recombinant production and/or the sequence information as to the coding sequence for the antibody may be retrieved and stored to permit subsequent synthesis of the appropriate nucleic acids. The availability of the information contained in the coding sequences and rapid synthesis and cloning techniques along with known methods of recombinant production permits rapid production of needed antibodies in the event of a pandemic or other emergency.

Applicants have recovered multiple monoclonal antibodies that are immunoreactive with HA₀ protein of influenza from multiple clades (SEQ ID NOS:9-23, 26-40, 42-56, and 59-73). Other sequences include the amino acid sequence for the human IgG1 heavy chain constant region (SEQ ID NO:8), the amino acid sequence for the human light chain constant kappa region (SEQ ID NO:24), the amino acid sequence for the human light chain constant lambda region (SEQ ID NO:25), the nucleotide sequence for the human heavy chain constant region (SEQ ID NO:41), the nucleotide sequence for the human light chain constant kappa region (SEQ ID NO:57), and the nucleotide sequence for the human light chain constant lambda region (SEQ ID NO:58).

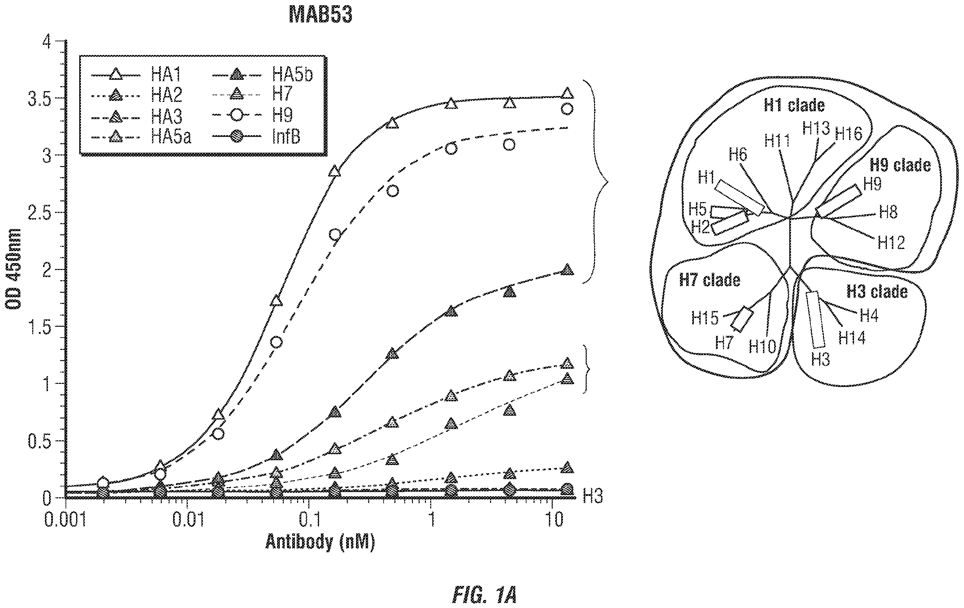

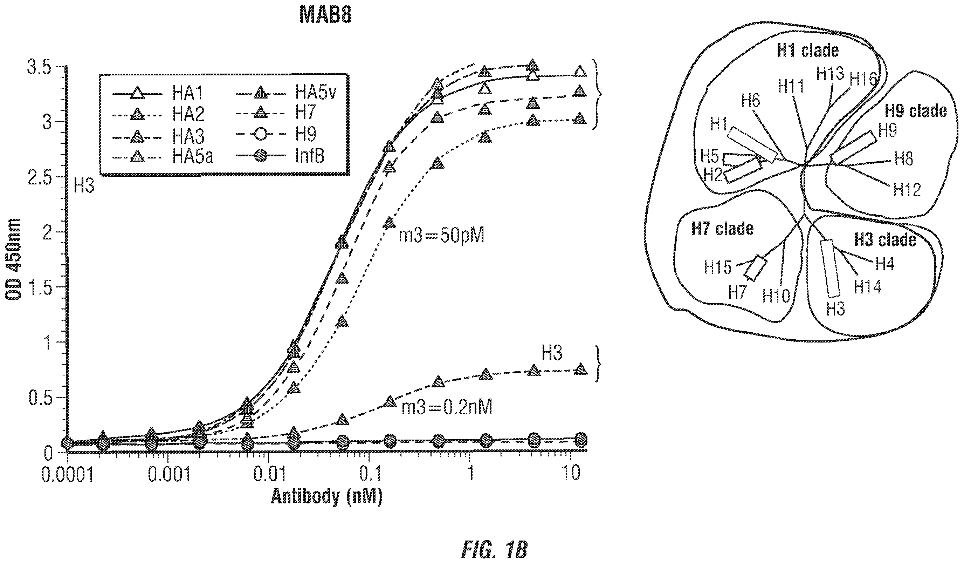

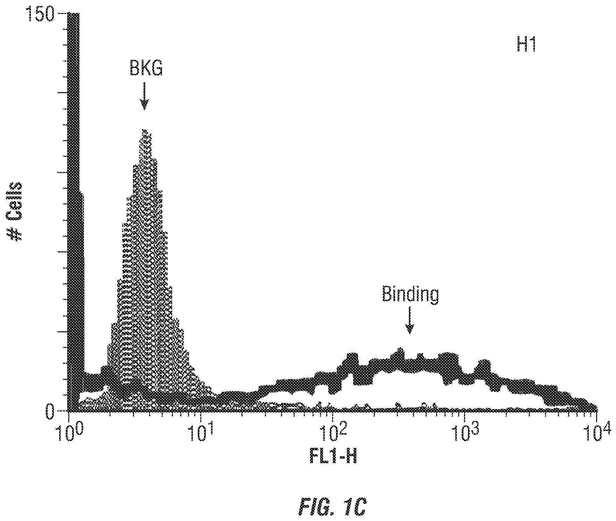

Two of these mAbs, MAB53 and MAB8, have substantial crossreactivity among important, distantly related influenza clades. As shown in Figures 1A and B, each of these binds to three different clades with reasonable or high affinity. MAB53 binds to HA₀ from the H1, H9 and H7 clades and MAB8 binds to HA₀ protein from H1, H7 and H3 clades. The results shown in Figure 1 were obtained by ELISA assay against HA₀ protein, and imply that the affinities are in the nanomolar range. Reactivity to native trimer of HA from all the Group 1 clades was verified using HA expressed in HEK293 cells with antibody binding measured by flow cytometry.

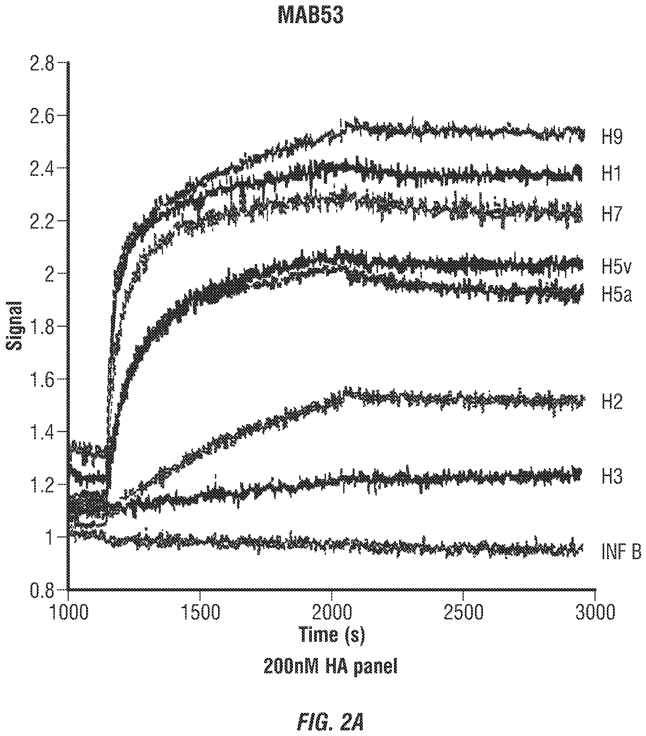

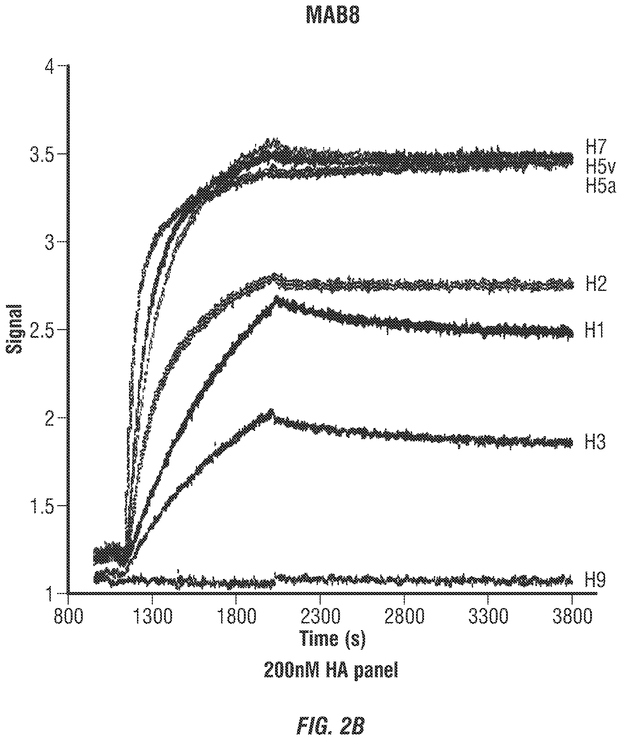

These results were confirmed using an alternative assay system, the biolevel interferometry based binding assay designated FortéBio® biosensor, as shown in Figures 2A and 2B. As measured by this more accurate assay, the affinities are as follows:
MAB53 / H1 = 60 pM, H5 = 6 nM, H7 = 70 pM, H9 = 30 pM;
MAB8 / H1 = 9 nM, H3 = 16 nM, H5 = 0.2 nM.

Both MAB53 and MAB8 are fully human antibodies, but similar antibodies characteristic of other species are also included in the invention. In the context of the invention, "antibodies" and their fragments include those portions of the molecule that are relevant for binding; thus, fragments would include variable regions only and "antibodies" as a general term would also be considered to include such fragments. Thus, F_{ab} fragments, F_{(ab')₂}, and Fv fragments are included as well as recombinantly produced single chain antibodies, and fusions of such constructs to create bispecific agents. Chimeric, humanized and human antibodies are all within the scope of the present invention as are antibody mimics based on other protein scaffolds such as fibronectin, transferrin, or lipocalin. Likewise, multiple technologies now exist for making a single antibody-like molecule that incorporates antigen specificity domains from two separate antibodies (bi-specific antibody). Thus, a single antibody with very broad strain reactivity can be constructed using the Fab domains of individual antibodies with broad reactivity to Group 1 and Group 2 respectively. Suitable technologies have been described by Macrogenics (Rockville, MD), Micromet (Bethesda, MD) and Merrimac (Cambridge, MA). (See, *e.g.,* Orcutt KD, Ackerman ME, Cieslewicz M, Quiroz E, Slusarczyk AL, Frangioni JV, Wittrup KD. A modular IgG-scFv bispecific antibody topology, Protein Eng Des Sel. (2010) 23:221-228; Fitzgerald J, Lugovskoy A. Rational engineering of antibody therapeutics targeting multiple oncogene pathways. MAbs. (2011) 1:3(3); Baeuerle PA, Reinhardt C. Bispecific T-cell engaging antibodies for cancer therapy. Cancer Res. (2009) 69:4941-4944.)

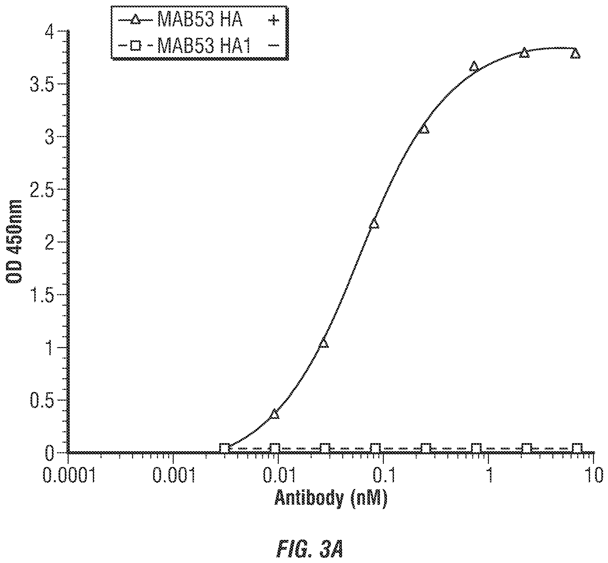

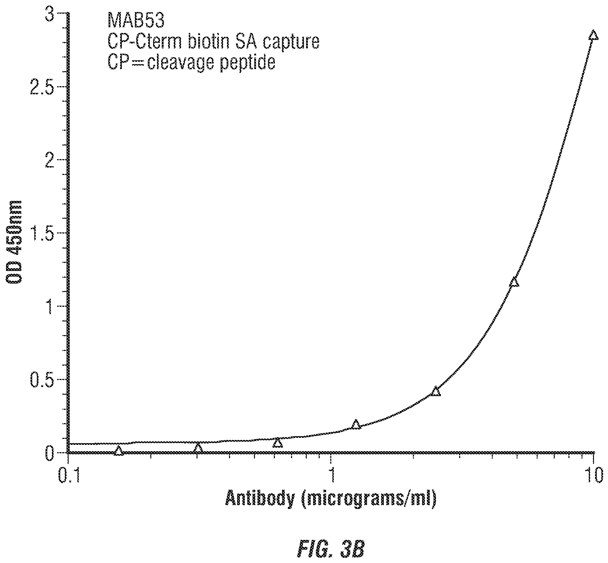

To identify the epitope to which MAB53 binds, ELISA assays were conducted with respect to uncleaved HA₀ protein, the HA₁ fragment, and the HA₂ fragment. As shown in Figures 3A and B, while MAB53 binds with high affinity to HA₀, it does not bind HA₁ implying binding to the complementary HA2 fragment. To confirm this hypothesis, a peptide derived from HA₂ was immobilized on a streptavidin coated plate using a C-terminal biotin. Specifically, the sequence tested was RGLFGAIAGFIENGW (SEQ ID NO:74). Irrelevant flanking portions were also used. MAB53 was confirmed as capable of binding to this peptide. As MAB53 does not bind to HA₀ when tested by Western blot, it is assumed that the dominant epitope is at least in part conformational in nature.

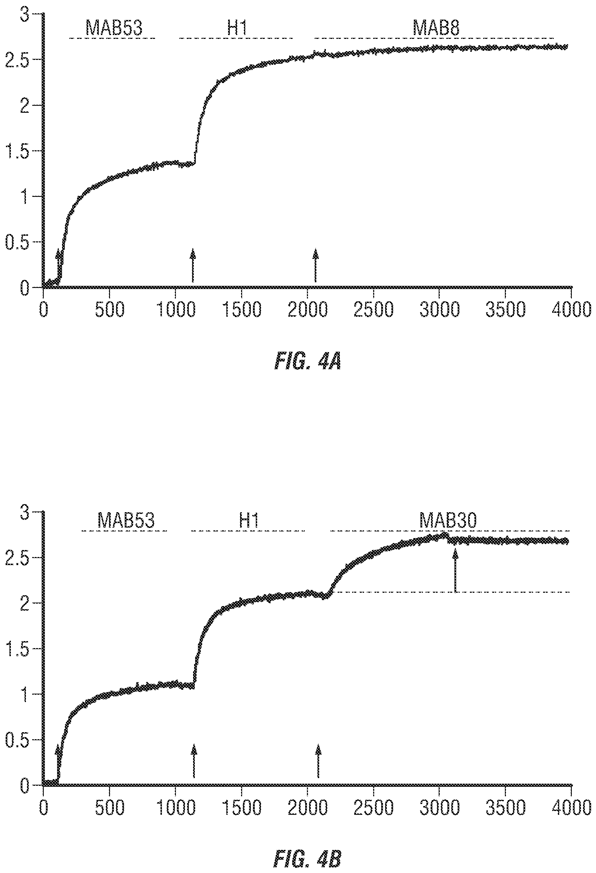

It has also been found that MAB8 and MAB53 bind to the same or nearby epitopes as demonstrated by their ability to compete with each other for binding to the HA₀ protein of the H1 clade. This was shown using a FortéBio® assay using 2 µg/ml of antibody and 50 nM HA₀ from H1. As shown in Figure 4A, the signal obtained from MAB53 bound to the FortéBio® surface is augmented when 50 nM HA₀ solution is added. However, when MAB8 is then added, no further signal occurs. Thus, MAB53 blocks the epitope bound by MAB8. As shown in Figure 4B, however, another antibody that is immunoreactive with HA₀, MAB30, binds, apparently, to a different epitope as the signal is enhanced when it is added to the coupled MAB53-HA₀.

Importantly, MAB53 and MAB8 differ in that MAB8 is released from the HA₀ protein when the pH is lowered to 6, whereas MAB53 is not. This difference is significant as this appears predictive of neutralizing capability. In tests for the ability of MAB8 to neutralize H1N1 viral infection in a plaque reduction assay in MDCK target cells, low doses of MAB53 of 1-5 µg/ml neutralized infection by H1N1, by H7N3, H5N1 and H9N2. However, MAB8 does not neutralize infection by these strains. Thus, neutralizing strains may be preferentially selected by washing bound MAB or fragment at pH 6 during the primary screen, thus removing from HA₀ MAB's that are unlikely to remain bound as the antibody-virus complex enters the cell via the endosomal compartment and thus will be expected to have reduced ability to neutralize the virus.

For example, in the CellSpot method HA₀ may be bound to solid support (fluorescent beads) and captured by the MAB or a mixture of MAB's, then washed at pH 6.

MAB53 is produced recombinantly and has been sequenced. The full-length sequences of the heavy chain and light chain are as follows:
Heavy Chain: **QVQLVQSGAEVRKPGSSVKVSCKVSGGIIRKYAINWVRQAPGQGLEWMGGIIAIFNTANYAQKFQGRVTITADESTSTVYMELSSLRSEDTAL**YYC**ARGMNYYSDYFDYWGQGSLVTVSP**ASTKGPSVFPLVPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID NO:75); and
Light Chain: **EIVLTQSPGTLSLSPGERATLSCRASQSVRSNNLAWYQHKPGQAPRLLIFGASSRATGIPDRFSGSGSGTDFTLTISRLEPEDFAVYYCQQYGSSPALTFGGGTKVEIK**RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO:76).

The bold sequences are variable domains, and the un-bolded sequences represent the IgG1 constant chain for the heavy chain and the kappa constant chain for the light chain.

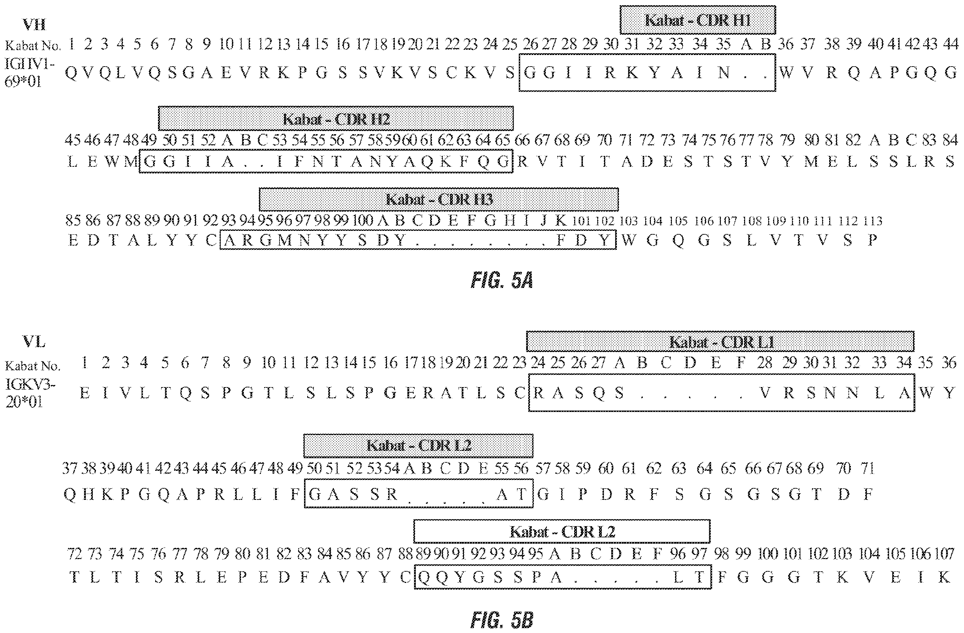

In addition, these variable regions have been analyzed according to the Kabat CDR assessment based on matching framework regions. As shown in Figure 5A, CDR1, CDR2, and CDR3 of the IGHV1-69*01 heavy chain (SEQ ID NO:83) are GGIIRKYAIN (SEQ ID NO:77), GGIIAIFNTANYAQKFQG (SEQ ID NO:78) and ARGMNYYSDYFDY (SEQ ID NO:79), respectively. As shown in Figure 5B, CDR1, CDR2, and CDR3 of the IGKV3-20*01 light chain (SEQ ID NO:84) are RASQSVRSNNLA (SEQ ID NO:80), GASSRAT (SEQ ID NO:81) and QQYGSSPALT (SEQ ID NO:82), respectively.

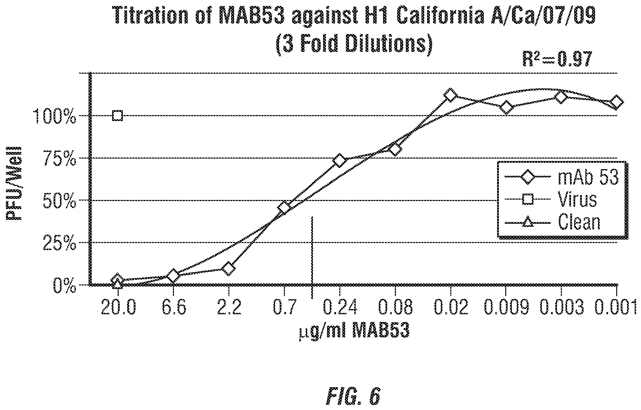

As shown in Figure 6, MAB53 neutralizes H1N1 *in vitro* in a plaque assay.

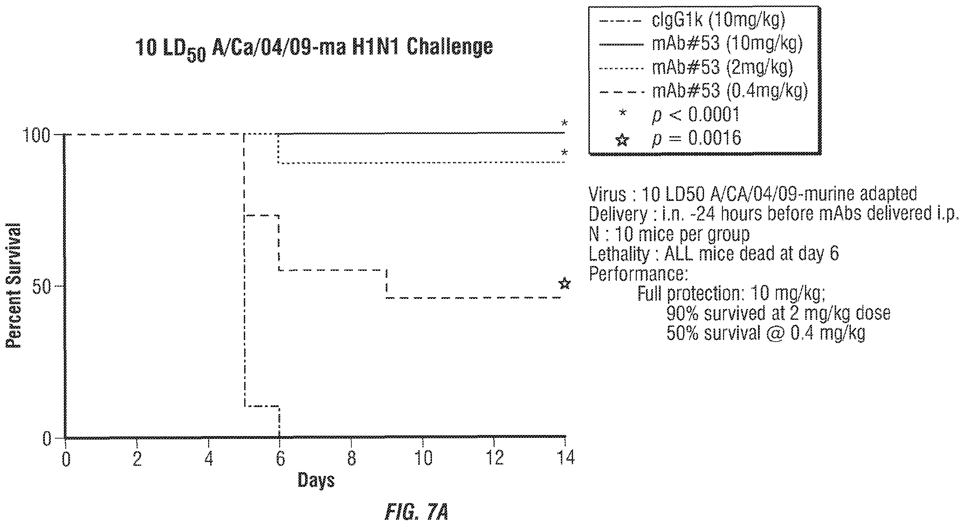

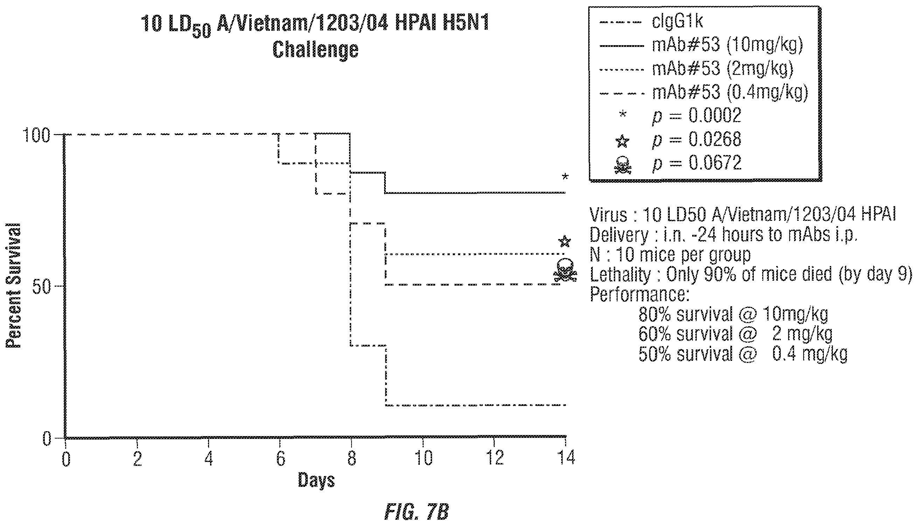

It has also been shown that mice pretreated with graded doses of MAB53 survive challenge with otherwise lethal titers of H1N1 and H5N1 viruses with 100% protection against H1N1 challenge, as shown in Figure 7. The potency is comparable to a prior art antibody described by Crucell which does not show activity against Group 2 strains. Throsby M., et al., PLoS One. (2008) 3:e3942. Epub 2008 Dec 16. These are heterosubtypic neutralizing monoclonal antibodies cross-protective against H5N1 and H1N1 recovered from human IgM+ memory B cells.

As shown in Figure 7A, MAB53 provided full protection at 10 mg/kg; 90% survived at 2 mg/kg and 50% survived at 0.4 mg/kg. In comparison, the prior art antibody from Crucell gave full protection at 2 mg/kg, but only 20% survived when 0.7 mg/kg were administered. This is despite the fact that the lethality of the viral dose was less than that in the experiment shown in Figure 7A; only 90% of the mice died after infection, whereas in the experiment shown in Figure 7A, all the mice died at day 6. This demonstrates that MAB53 is highly potent.

Where challenge by H5N1 was substituted for challenge by H1N1, for MAB53 shown in Figure 7B, 10 mg/kg gave 80% survival; 2 mg/kg gave 60% survival and 0.4 mg/kg gave 50% survival. In comparison, for the prior art antibody, 100% survival was obtained at 5 mg/kg and 60% survival at 1.7 mg/kg. Thus, the survival rates at 1.7 mg/kg and 2 mg/kg were comparable. In this case, the viral dose itself was slightly less potent in the mice tested with MAB53.

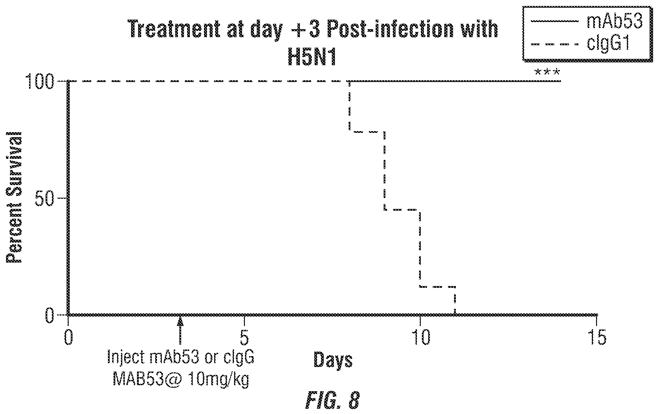

As shown in Figure 8, MAB53 (10 mg/kg) was administered as a post-infection treatment at day +3 against the high pathology H5N1 strain. The control antibody is isotype matched but does not recognize any flue antigen. The infection and treatment protocol is the same as that for Figure 7A, but given at day +3 instead of day -1.

Pepscan analysis was performed, establishing that MAB53 and CR6261 bind to similar regions of HA, but different epitopes (data not shown). This is consistent with the different activity of the two antibodies.

Thus, MAB53 and antibodies that bind to the same epitope under the same conditions are effective as passive vaccines suitable for protection of populations against epidemics and pandemics, and for prophylactic or therapeutic use against seasonal influenza for patients with a weakened immune system.

### SEQUENCE LISTING

NVPEKQTR (SEQ ID NO:1)
GIFGAIAGFIE (SEQ ID NO:2)
NIPSIQSR (SEQ ID NO:3)
GLFGAIAGFIE (SEQ ID NO:4)
PAKLLKER (SEQ ID NO:5)
GFFGAIAGFLE (SEQ ID NO:6)
RGI/L/F FGAIAGFLE (SEQ ID NO:7).

Human IgG1 HC amino acid sequence of constant region (SEQ ID NO:8)
MAB1 HC amino acid sequence of variable domain (SEQ ID NO:9)
MAB8 HC amino acid sequence of variable domain (SEQ ID NO: 10)
MAB30 HC amino acid sequence of variable domain (SEQ ID NO:11)
MAB42 HC amino acid sequence of variable domain (SEQ ID NO: 12)
MAB48 HC amino acid sequence of variable domain (SEQ ID NO: 13)
MAB49 HC amino acid sequence of variable domain (SEQ ID NO: 14)
MAB52 HC amino acid sequence of variable domain (SEQ ID NO: 15)
MAB53 HC amino acid sequence of variable domain (SEQ ID NO:16)
MAB285 HC amino acid sequence of variable domain (SEQ ID NO:17)
MAB321 HC amino acid sequence of variable domain (SEQ ID NO:18)
MAB322 HC amino acid sequence of variable domain (SEQ ID NO:19)
MAB375 HC amino acid sequence of variable domain (SEQ ID NO:20)
MAB376 HC amino acid sequence of variable domain (SEQ ID NO:21)
MAB377 HC amino acid sequence of variable domain (SEQ ID NO:22)
MAB378 HC amino acid sequence of variable domain (SEQ ID NO:23)
Human LC amino acid sequence of constant kappa region (SEQ ID NO:24)
Human LC amino acid sequence of constant lambda region (SEQ ID NO:25)
MAB1 LC amino acid sequence (SEQ ID NO:26)
MAB8 LC amino acid sequence (SEQ ID NO:27)
MAB30 LC amino acid sequence (SEQ ID NO:28)
MAB42 LC amino acid sequence (SEQ ID NO:29)
MAB48 LC amino acid sequence (SEQ ID NO:30)
MAB49 LC amino acid sequence (SEQ ID NO:31)
MAB52 LC amino acid sequence (SEQ ID NO:32)
MAB53 LC amino acid sequence (SEQ ID NO:33)
MAB285 LC amino acid sequence (SEQ ID NO:34)
MAB321 LC amino acid sequence (SEQ ID NO:35)
MAB322 LC amino acid sequence (SEQ ID NO:36)
MAB375 LC amino acid sequence (SEQ ID NO:37)
MAB376 LC amino acid sequence (SEQ ID NO:38)
MAB377 LC amino acid sequence (SEQ ID NO:39)
MAB378 LC amino acid sequence (SEQ ID NO:40)
Human IgG1 HC nucleotide sequence of constant region (introns are underlined) (SEQ ID NO:41)
MAB1 HC variable domain nucleotide sequence (SEQ ID NO:42)
MAB8 HC variable domain nucleotide sequence (SEQ ID NO:43)
MAB30 HC variable domain nucleotide sequence (SEQ ID NO:44)
MAB42 HC variable domain nucleotide sequence (SEQ ID NO:45)
MAB48 HC variable domain nucleotide sequence (SEQ ID NO:46)
MAB49 HC variable domain nucleotide sequence (SEQ ID NO:47)
MAB52 HC variable domain nucleotide sequence (SEQ ID NO:48)
MAB53 HC variable domain nucleotide sequence (SEQ ID NO:49)
MAB285 HC variable domain nucleotide sequence (SEQ ID NO:50)
MAB321 HC variable domain nucleotide sequence (SEQ ID NO:51)
MAB322 HC variable domain nucleotide sequence (SEQ ID NO:52)
MAB375 HC variable domain nucleotide sequence (SEQ ID NO:53)
MAB376 HC variable domain nucleotide sequence (SEQ ID NO:54)
MAB377 HC variable domain nucleotide sequence (SEQ ID NO:55)
MAB378 HC variable domain nucleotide sequence (SEQ ID NO:56)
Human LC nucleotide sequence of constant kappa region (SEQ ID NO:57)
Human LC nucleotide sequence of constant lambda region (SEQ ID NO:58)
MAB1 LC variable domain nucleotide sequence (SEQ ID NO:59)
MAB8 LC variable domain nucleotide sequence (SEQ ID NO:60)
MAB30 LC variable domain nucleotide sequence (SEQ ID NO:61)
MAB42 LC variable domain nucleotide sequence (SEQ ID NO:62)
MAB48 LC variable domain nucleotide sequence (SEQ ID NO:63)
MAB49 LC variable domain nucleotide sequence (SEQ ID NO:64)
MAB52 LC variable domain nucleotide sequence (SEQ ID NO:65)
MAB53 LC variable domain nucleotide sequence (SEQ ID NO:66)
MAB285 LC variable domain nucleotide sequence (SEQ ID NO:67)
MAB321 LC variable domain nucleotide sequence (SEQ ID NO:68)
MAB322 LC variable domain nucleotide sequence (SEQ ID NO:69)
MAB375 LC variable domain nucleotide sequence (SEQ ID NO:70)
MAB376 LC variable domain nucleotide sequence (SEQ ID NO:71)
MAB377 LC variable domain nucleotide sequence (SEQ ID NO:72)
MAB378 LC variable domain nucleotide sequence (SEQ ID NO:73)
RGLFGAIAGFIENGW (SEQ ID NO:74).
MAB53 Heavy Chain (SEQ ID NO:75)
MAB53 Light Chain (SEQ ID NO:76)
GGIIRKYAIN (SEQ ID NO:77)
GGIIAIFNTANYAQKFQG (SEQ ID NO:78)
ARGMNYYSDYFDY (SEQ ID NO:79)
RASQSVRSNNLA (SEQ ID NO:80)
GASSRAT (SEQ ID NO:81)
QQYGSSPALT (SEQ ID NO:82)
IGHV1-69*01 (SEQ ID NO:83)
IGKV3-20*01 (SEQ ID NO:84)

## Claims

1. A human monoclonal antibody, optionally a bispecific antibody, or immunoreactive fragment thereof, comprising a heavy chain comprising CDR1 of the sequence GGIIRKYAIN (SEQ ID NO:77), CDR2 of the sequence GGIIAIFNTANYAQKFQG (SEQ ID NO:78), and CDR3 of the sequence ARGMNYYSDYFDY (SEQ ID NO:79) and a light chain comprising CDR1 of the sequence RASQSVRSNNLA (SEQ ID NO:80), a CDR2 of the sequence GASSRAT (SEQ ID NO:81), and a CDR3 of the sequence QQYGSSPALT (SEQ ID NO:82), wherein the antibody or fragment binds to HA₀ and HA₂ protein stalk region from influenza viral clades that include at least one clade from each of Group 1 and Group 2 of influenza A.

2. The antibody or fragment of claim 1 that binds to HA₀ protein stalk region from influenza A viral clades H1, H7 and H9 or with influenza A viral clades H1, H7 and H3.

3. The antibody or fragment of claim 1, wherein the antibody or fragment remains bound to influenza HA0 protein when the pH is lowered to 6 *in vitro.*

4. The antibody or fragment of any of claims 1 - 3 that remains bound to the virus following uptake via the endosomal pathway.

5. The antibody or fragment of any of claims 1 - 4 that neutralizes infection by H1N1, H7N3 or H5N1 virus in MDCK cells.

6. The antibody or fragment of any of claims 1-5 that is protective in mice against challenge with otherwise lethal titers of H1N1 or H5N1 at a single dose of 1-10 mg/kg.

7. The antibody or fragment of claim 1 which is an antibody or fragment which comprises a heavy chain comprising the sequence
QVQLVQSGAEVRKPGSSVKVSCKVSGGIIRKYAINWVRQAPGQGLEWMGG IIAIFNTANYAQKFQGRVTITADESTSTVYMELSSLRSEDTALYYCARGMNYYSDY FDYWGQGSLVTVSP (amino acids 1-120 of SEQ ID NO:75).

8. The antibody or fragment of claim 1 or 7 which comprises a light chain comprising the sequence
EIVLTQSPGTLSLSPGERATLSCRASQSVRSNNLAWYQHKPGQAPRLLIFGA SSRATGIPDRFSGSGSGTDFTLTISRLEPEDFAVYYCQQYGSSPALTFGGGTKVEIK (amino acids 1-109 of SEQ ID NO:76).

9. A pharmaceutical composition comprising the antibody or fragment of any of claims 1-8.

10. The antibody or fragment of any of claims 1-8 or the composition of claim 9 for use in a method for the treatment or prophylaxis of influenza infection in a subject.

11. A recombinant expression system that comprises a nucleotide sequence encoding the heavy chain and a nucleotide sequence encoding the light chain variable region of the antibody or fragment of any of claims 1, 7, or 8 operably linked to control sequences for expression.

12. Recombinant host cells modified to contain the expression system of claim 11.

13. A method to produce a monoclonal antibody or fragment immunoreactive with influenza virus which method comprises culturing the cells of claim 12 under conditions wherein said nucleotide sequence is expressed.

## Patentansprüche

1. Humaner monoklonaler Antikörper, optional ein bispezifischer Antikörper, oder ein immunreaktives Fragment davon, umfassend eine schwere Kette umfassend CDR1 der Sequenz GGIIRKYAIN (SEQ ID Nr:77), CDR2 der Sequenz GGIIAIFNTANYAQKFQG (SEQ ID Nr:78), und CDR3 der Sequenz ARGMNYYSDYFDY (SEQ ID Nr:79) und eine leichte Kette umfassend CDR1 der Sequenz RASQSVRSNNLA (SEQ ID Nr:80), CDR2 der Sequenz GASSRAT (SEQ ID Nr:81), and CDR3 der Sequenz QQYGSSPALT (SEQ ID Nr:82), wobei der Antikörper oder das Fragment an das HA₀ und HA₂ Protein der Stielregion von Influenza viralen Kladen bindet, die mindestens eine Klade von jeder von Gruppe 1 und Gruppe 2 von Influenza A einschließt.

2. Antikörper oder Fragment nach Anspruch 1, der/das an das H₀ Protein der Stielregion von Influenza A viralen Kladen H1, H7 und H9 bindet oder an Influenza A virale Kladen H1, H7 und H3.

3. Antikörper oder Fragment nach Anspruch 1, wobei der Antikörper oder das Fragment an das Influenza HA₀ Protein gebunden bleibt, wenn der pH auf 6 *in vitro* erniedrigt wird.

4. Antikörper oder Fragment nach einem der Ansprüche 1 - 3, der/das an das Virus gebunden bleibt nach Aufnahme über den endosomalen Pfad.

5. Antikörper oder Fragment nach einem der Ansprüche 1 - 4, der/das die Infektion durch H1N1, H7N3 oder H5N1 Virus in MDCK-Zellen neutralisiert.

6. Antikörper oder Fragment nach einem der Ansprüche 1 - 5, der/das in Mäusen gegen Probleme mit andernfalls letalen Titern von H1N1 oder H5N1 in einer einzigen Dosis von 1-10 mg/kg schützend ist.

7. Antikörper oder Fragment nach Anspruch 1, der/das ein Antikörper oder Fragment ist, der/das eine schwere Kette umfasst, umfassend die Sequenz
QVQLVQSGAEVRKPGSSVKVSCKVSGGIIRKYAINWVRQAPGQGLEWMGG IIAIFNTANYAQKFQGRVTITADESTSTVYMELSSLRSEDTALYYCARGMNYYSDY FDYWGQGSLVTVSP (Aminosäuren 1-120 von SEQ ID Nr:75).

8. Antikörper oder Fragment nach Anspruch 1 oder 7, der/das eine leichte Kette umfasst, umfassend die Sequenz
EIVLTQSPGTLSLSPGERATLSCRASQSVRSNNLAWYQHKPGQAPRLLIFGA SSRATGIPDRFSGSGSGTDFTLTISRLEPEDFAVYYCQQYGSSPALTFGGGTKVEIK (Aminosäuren 1-109 von SEQ ID Nr:76).

9. Pharmazeutische Zusammensetzung umfassend den Antikörper oder das Fragment nach einem der Ansprüche 1-8.

10. Antikörper oder Fragment nach einem der Ansprüche 1 - 8 oder Zusammensetzung nach Anspruch 9 zur Verwendung in einem Verfahren zur Behandlung oder Prophylaxe von Influenzainfektionen in einem Subjekt.

11. Rekombinantes Expressionssystem, das eine Nukleotidsequenz umfasst, kodierend die schwere Kette und eine Nukleotidsequenz kodierend die leichte Kette variable Region des Antikörpers oder Fragments nach einem der Ansprüche 1, 7 oder 8, funktionsfähig verbunden mit Kontrollsequenzen für Expression.

12. Rekombinante Wirtszellen, modifiziert, um das Expressionssystem zu beinhalten.

13. Verfahren zum Herstellen eines monoklonalen Antikörpers oder Fragments, immunreaktiv mit Influenzavirus, wobei das Verfahren Kultivieren der Zellen nach Anspruch 12 unter Bedingungen, unter denen die Nukleotidsequenz exprimiert wird, umfasst.

## Revendications

1. Anticorps monoclonal humain, facultativement un anticorps bispécifique, ou fragment immunoréactif de celui-ci, comprenant une chaîne lourde comprenant une CDR1 de la séquence GGIIRKYAIN (SEQ ID NO:77), une CDR2 de la séquence GGIIAIFNTANYAQKFQG (SEQ ID NO:78), et une CDR3 de la séquence ARGMNYYSDYFDY (SEQ ID NO: 79) et une chaîne légère comprenant une CDR1 de la séquence RASQSVRSNNLA (SEQ ID NO: 80), une CDR2 de la séquence GASSRAT (SEQ ID NO: 81), et une CDR3 de la séquence QQYGSSPALT (SEQ ID NO: 82), où l'anticorps ou le fragment se lie à la région de tige de la protéine HA₀ ou HA₂ de clades du virus de la grippe qui incluent au moins un clade de chacun du groupe 1 et du groupe 2 de la grippe A.

2. Anticorps ou fragment selon la revendication 1 qui se lie à la région de tige de la protéine HA₀ des clades H1, H7 et H9 du virus de la grippe A ou avec les clades H1, H7 et H3 du virus de la grippe A.

3. Anticorps ou fragment selon la revendication 1, où l'anticorps ou le fragment reste lié à la protéine HA0 de la grippe lorsque le pH est réduit à 6 *in vitro.*

4. Anticorps ou fragment selon l'une quelconque des revendications 1 à 3 qui reste lié au virus suite à une absorption via la voie endosomale.

5. Anticorps ou fragment selon l'une quelconque des revendications 1 à 4 qui neutralise une infection par un virus H1N1, H7N3 ou H5N1 dans des cellules MDCK.

6. Anticorps ou fragment selon l'une quelconque des revendications 1 à 5 qui confère une protection chez des souris contre une infection avec des titres autrement létaux de H1N1 ou H5N1 à une dose unique de 1-10 mg/kg.

7. Anticorps ou fragment selon la revendication 1 qui est un anticorps ou un fragment qui comprend une chaîne lourde comprenant la séquence
QVQLVQSGAEVRKPGSSVKVSCKVSGGIIRKYAINWVRQAPGQGLEWMGGIIAIF NTANYAQKFQGRVTITADESTSTVYMELSSLRSEDTALYYCARGMNYYSDYFDYWGQG SLVTVSP (acides aminés 1-120 de SEQ ID NO: 75).

8. Anticorps ou fragment selon la revendication 1 ou 7 qui comprend une chaîne légère comprenant la séquence
EIVLTQSPGTLSLSPGERATLSCRASQSVRSNNLAWYQHKPGQAPRLLIFGASSR ATGIPDRFSGSGSGTDFTLTISRLEPEDFAVYYCQQYGSSPALTFGGGTKVEIK (acides aminés 1-109 de SEQ ID NO: 76).

9. Composition pharmaceutique comprenant l'anticorps ou le fragment selon l'une quelconque des revendications 1 à 8.

10. Anticorps ou fragment selon l'une quelconque des revendications 1 à 8 ou composition selon la revendication 9 destiné(e) à être utilisé(e) dans un procédé de traitement ou de prophylaxie d'une infection par la grippe chez un sujet.

11. Système d'expression recombinant qui comprend une séquence de nucléotides codant pour la chaîne lourde et une séquence de nucléotides codant pour la région variable de chaîne légère de l'anticorps ou du fragment selon l'une quelconque des revendications 1, 7, ou 8, en liaison fonctionnelle avec des séquences de contrôle pour l'expression.

12. Cellules hôtes recombinantes modifiées pour contenir le système d'expression selon la revendication 11.

13. Procédé pour produire un anticorps monoclonal ou un fragment immunoréactif avec un virus de la grippe, lequel procédé comprend la mise en culture des cellules selon la revendication 12 dans des conditions dans lesquelles ladite séquence de nucléotides est exprimée.
